Europäisches Patentamt

European Patent Office

Office européen des brevets

(19)

(11) Publication number: **0 131 624 B1**

# EUROPEAN PATENT SPECIFICATION

(12)

(45) Date of publication of patent specification: **16.09.92**

(51) Int. Cl.5: **C12N 15/82**

(21) Application number: **84900783.6**

(22) Date of filing: **16.01.84**

(86) International application number:
**PCT/US84/00049**

(87) International publication number:
**WO 84/02919 (02.08.84 84/18)**

The file contains technical information submitted after the application was filed and not included in this specification

(54) PLASMIDS FOR TRANSFORMING PLANT CELLS.

(30) Priority: **17.01.83 US 458411**

(43) Date of publication of application:
**23.01.85 Bulletin 85/04**

(45) Publication of the grant of the patent:
**16.09.92 Bulletin 92/38**

(84) Designated Contracting States:
**AT BE CH DE FR GB LI LU NL SE**

(56) References cited:
**EP-A- 0 116 718**
**EP-A- 0 122 791**
**WO-A-84/02920**

**Nature 287 (1980) 654-656**

**EMBO J. 1 (1982) 147 - 152**

(73) Proprietor: **Monsanto Company
Patent Department 800 North Lindbergh Boulevard
St. Louis Missouri 63167-7020(US)**

(72) Inventor: **FRALEY, Robert, Thomas
828 Lisakay Drive
Glendale, MO 63167(US)**
Inventor: **ROGERS, Stephen, Gary
312 Sylvester Avenue
Webster Groves, MO 63119(US)**

(74) Representative: **Ernst, Hubert et al
Monsanto Services International S.A., Patent
Department, Avenue de Tervuren 270-272,
Letter Box No. 21
B-1150 Brussels(BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

PROCEEDINGS OF THE NATIONAL ACADEMY OF SCIENCES OF THE USA, vol. 80, no. 15, August 1983, pages 4803-4807, Washington, US; R.T. FRALEY et al.: "Expression of bacterial genes in plant cells"

CHEMICAL ABSTRACTS, vol. 97, 25th October 1982, page 182, abstract 139517v, Columbus, Ohio, US; G.M.S. VAN SLOGTEREN et al.: "The lysopine dehydrogenase gene used as a marker for the selection of octopine crown gall cells", & PLANT MOL. BIOL. 1982, 1(2), 133-42

CHEMICAL ABSTRACTS, vol. 101, no. 3, July 1984, page 176, abstract 18452n, Columbus, Ohio, US; J.D. KEMP et al.: "Transfer of a functional gene via the Ti plasmid", & CURR: TOP. PLANT BIOCHEM. PHYSIOL., PROC. INAUG. PLANT BIOCHEM. PHYSIOL. SYMP. 1982 (Publ. 1983), 1, 170-9

CHEMICAL ABSTRACTS, vol. 99, 26th September 1983, page 182, abstract 100292j, Columbus, Ohio, US; L COMAI et al.: "A new technique for genetic engineering of Agrobacterium Ti plasmid", & PLASMID 1983, 10(1), 21-30

NATURE, vol. 276, 7th December 1978, pages 633-634, Macmillan Journals Ltd.; J.E. BERINGER et al.: "Transfer of the drug-resistance transposon Tn5 to Rhizobium"

F. AHMAD et al.: "From genetoprotein; trans-lation into biotechnology", Miami Winter Symposia, vol. 19, 1982, page 514, Academic Press Inc., New York, US; P.V. CHOUDARY et al.: "Studies on expression of the phaseolin gene of french bean seeds in sunflower plant cells"

STADLER SYMP., vol. 13, 1981, University of Missouri, Columbia, pages 39-51; M.-D. CHILTON et al.: "Tailoring the agrobacterium Ti plasmid as a vector for plant genetic engineering"

Schell et al., Genetic Engineering to Biotechnology - The Critical Transition, Edited by W.J. Whelan and Sandra Black, Published by John Wiley & Sons Ltd., pages 41-52 (1982)

Zambryski et al., Journal of Molecular and Applied Genetics, vol. 1, No. 4 pages 361-370(1982)

Matzke et al., Journal of Molecular and Applied Genetics, Vol. 1, No. 1, pages 39 - 49 (1981)

Leemans et al., Journal of Molecular and Applied Genetics, Vol. 1, No. 2, pages 149 - 164 (1981)

Leeemans et al., Molecular Biology of Plant Tumors, edited by G. Kahl and J.S. Schell, published by Academic Press Inc., pages 537 - 545 (1982)

## Description

Technical Field

This invention is in the fields of genetic engineering, plant biology, and bacteriology.

## BACKGROUND ART

In the past decade, the science of genetic engineering has developed rapidly. A variety of processes are known for inserting a heterologous gene into bacteria, whereby the bacteria become capable of efficient expression of the inserted genes. Such processes normally involve the use of plasmids which may be cleaved at one or more selected cleavage sites by restriction endonucleases. Typically, a gene of interest is obtained by cleaving one piece of DNA, and the resulting DNA fragment is mixed with a fragment obtained by cleaving a vector such as a plasmid. The different strands of DNA are then connected ("ligated") to each other to form a reconstituted plasmid. See, for example, U.S. Patents 4,237,224 (Cohen and Boyer, 1980); 4,264,731 (Shine, 1981); 4,273,875 (Manis, 1981); 4,322,499 (Baxter et al, 1982), and 4,336,336 (Silhavy et al, 1982).

A variety of other reference works are available. Some of these works describe the natural process whereby DNA is transcribed into mRNA and mRNA is translated into protein, see, e.g., L. Stryer, Biochemistry, 2nd edition, p 559 et seq. (W. H. Freeman and Co., 1981); A. L. Lehninger, Biochemistry, 2nd edition, p. 853 et seq. (Worth Publ., 1975). Other works describe methods and products of genetic manipulation; see, e.g., T. Maniatis et al, Molecular Cloning, A Laboratory Manual (Cold Spring Harbor Labs, 1982); J.K. Setlow and A. Hollaender, Genetic Engineering, Principles and Methods (Plenum Press, 1979). Hereafter, all references will be cited in abbreviated form; a list of complete citations is included after the Examples.

Most of the genetic engineering work performed to date involves the insertion of genes into various types of cells, primarily bacteria such as E. coli, various other microorganisms such as yeast, and mammalian cells. However, many of the techniques and substances used for genetic engineering of animal cells and microorganisms are not directly applicable to genetic engineering involving plants.

As used herein, the term "plant" refers to a multicellular differentiated organism that is capable of photosynthesis, such as angiosperms and multicellular algae. This does not include microorganisms, such as bacteria, yeast, and fungi. The term "plant cell" includes any cell derived from a plant; this includes undifferentiated tissue such as callus or crown gall tumor, as well as plant seeds, propagules, pollen, or plant embryos.

Ti and Ri Plasmids

The tumor-inducing (Ti) plasmid of Agrobacterium tumefaciens has been proposed for use as a natural vector for introducing foreign genetic information into plant cells (Hernalsteen et al 1980; Rorsch and Schilperoort, 1978). Certain types of A. tumefaciens are capable of infecting a wide variety of plant cells, causing crown gall disease. The process of infection is not fully understood. At least part of the Ti plasmid enters the plant cell. Various metabolic alterations occur, and part of the Ti plasmid is inserted into the genome of the plant (presumably into the chromosomes). The part of the Ti plasmid that enters the plant genome is designated as "transferred DNA" (T-DNA). T-DNA is stably maintained in the plant DNA (Chilton et al, 1977; Yadev et al, 1980; Willmitzer et al, 1980; Otten et al, 1981).

Research by several laboratories has led to the characterization of several structural (i.e., protein coding) genes located in T-DNA (Garfinkel et al 1981; Leemans et al 1982), as well as other DNA sequences which appear to serve various other functions. For example, two sequences referred to as the "left border" and the "right border" appear to delineate T-DNA and may be involved in the process whereby T-DNA is transferred into plant chromosomes (Zambryski et al 1982).

A different species of Agrobacterium, A. rhizogenes, carries a "root-inducing" (Ri) plasmid which is similar to the Ti plasmid. Infection of a plant cell by A. rhizogenes causes hairy root disease. Like the Ti plasmid, a segment of DNA called "T-DNA" (also referred to by some researchers as "R-DNA") is transferred into the plant genome of an infected cell.

Various other bacteria are also reported to be capable of causing genetic transformation of plant cells, including A. rubi and certain bacteria of the genus Rhizobia which have been treated with a mutagenic agent. Hooykaas et al, at page 156 of Setlow and Holaender, 1979.

As used herein, the term "Ti plasmid" includes any plasmid (1) which is contained in a microorganism, other than a virus, which is capable of causing genetic transformation of one or more types of plants or plant cells, and (2) which contains a segment of DNA which is inserted into a plant genome. This includes Ri plasmids.

As used herein, the term "T-DNA" refers to a segment of DNA in or from a Ti plasmid (1) which has been inserted into the genome of one or more types of plant cells, or (2) which is contained in a segment of DNA that is located between two sequences of bases which are capable of serving as T-DNA borders. As used herein, the terms "T-DNA border" and "border" are determined and applied empirically; these terms shall refer to a sequence of bases which appears at or near the end of a segment of DNA which is transferred from a Ti plasmid into a plant genome.

Despite the existing knowledge of T-DNA and Ti plasmids, no one prior to this invention has been able to utilize these vectors for the introduction of foreign genes which are expressed in genetically modified plants. A variety of obstacles to such use have been encountered in genetic engineering efforts. Such obstacles include:

1) the large size (approximately 200,000 base pairs) and resulting complexity of Ti plasmids preclude the use of standard recombinant DNA techniques to genetically modify and/or insert foreign genes into specific sites in the T-DNA. For example, there are no known unique restriction endonuclease cleavage sites in a Ti plasmid (Leemans et al, 1982).

2) the T-DNA, which is inserted into and expressed in plant cells, contains genes which are involved in the production of high levels of phytohormones in the transformed plant cells (Leemans et al 1982). The high levels of phytohormones interfere with the normal metabolic and regenerative process of the cells, and prevent the formation of phenotypically normal plants from the cells (Braun and Wood, 1976; Yang et al, 1980). Exceptions to this are rare cases where the T-DNA has undergone extensive spontaneous deletions in planta to eliminate those genes involved in phytohormone production. Under these conditions, normal plants are reported to be obtainable at low frequency (Otten et al, 1981). However, the T-DNA genes involved in phytohormone production could not be deleted prior to this invention, since they were very important in the identification and/or selection of transformed plant cells (Marton et al, 1979).

As described above, simple recombinant DNA techniques for introducing foreign genes into plasmids are not applicable to the large Ti plasmid. As a result, several indirect methods have been developed and are discussed below. The first reported use of the Ti plasmid as a vector was in model experiments in which bacterial transposons were inserted into T-DNA and subsequently introduced into plant cells. The bacterial transposons were reported to be stably maintained in the plant genome (Hernalsteens et al, 1980; Garfinkel et al 1981). However, in these cases the transformed tumor tissues were found to be incapable of regeneration into normal plants, and there was no reported evidence for the expression of bacterial genes in the plant cells. In addition, because the insertion of bacterial transposons is believed to be essentially random, a great deal of effort was required to identify and localize the position of the inserted DNA in these examples.

In later experiments, J. Leemans, H. de Greve et al (1982) demonstrated the expression of dihydrofolate reductase, a characteristic product of the bacterial Tn 7 transposon, by crown gall tobacco cells that had been transformed by mutant Ti plasmids containing the Tn 7 transposon sequence in the T-DNA. The authors speculate that it should be possible to insert, behind the T-DNA promotors for opine synthesis, the sequences of bacterial genes that code for functions that inactivate other antibiotics. Thus they suppose it might be possible to achieve good expression and possibly sufficient antibiotic resistance to allow for direct selection of the transformed plant cells.

Other researchers have reported the use of intermediate vectors which replicate in both E.coli and A. tumefaciens (Matzke and Chilton, 1981; Leemans et al 1981; Garfinkel et al, 1981). The intermediate vectors contain relatively small subfragments of the Ti plasmid which can be manipulated using standard recombinant DNA techniques. The subfragments can be modified by the deletion of specific sequences or by the insertion of foreign genes at specific sites. The intermediate vectors containing the modified T DNA subfragment are then introduced into A. tumefaciens by transformation or conjugation. Double recombination between the modified T-DNA fragment on the intermediate vector and its wild-type counterpart on the Ti plasmid results in the replacement of the wild-type copy with the modified fragment. Cells which contain the recombined Ti plasmids can be selected using appropriate antibiotics.

Various foreign DNA's have been inserted at specific sites in the T-DNA by this method and they have been reported to be stably transferred into plant cells (Matzke and Chilton, 1981, Leemans et al 1981, 1982). However, such foreign genes have not been reported to be capable of expression in plant cells. Possible reasons for this are discussed by M-D Chilton et al (1981). For example it is suggested that the length and sequence of the 5'- untranslated portion of the transcript could affect the efficiency of capping, splicing, transport to the cytoplasm or initiation of translation. Furthermore, in the procedure described above, it is preferred for a double crossover event to occur, in order to substitute the modified DNA fragment for the wild-type copy. A single crossover results in the formation of a co-integrate plasmid which contains two copies of the T-DNA subfragments. This duplication is undesirable in these methods since homologous recombination, which can occur in A. tumefaciens cells or in plant cells, can result in the loss of the inserted foreign gene(s).

A major disadvantage of the above methods is that the frequency of double recombination is quite low, about $10^{-4}$ to $10^{-9}$ (Leemans et al, 1981) and it requires extensive effort to identify and isolate the rare double-crossover recombinants. As a result, the number and types of experiments which can be performed using existing methods for genetically engineering the Ti plasmid is severely limited.

Choudary et al, (1982) using a similar procedure, report the transfer of the gene for phaseolin from bean to sunflower plant cells, using recombinant cells of A. tumefaciens as vectors. Transcription of the phaseolin gene in the transformed sunflower cells was reported, but the production of phaseolin protein could not be detected.

Another report on the prospects of using the Ti plasmids of Agrobacteria as vectors for the genetic manipulation of plants is that of Ream and Gordon (1982).

EP-A-0 116 718 which is prior art relative to the present application under Article 54(3) EPC, describes modified acceptor Ti plasmids which allow the introduction of any gene(s) of interest contained within an intermediate cloning vector carrying a region of homology with a corresponding region in the acceptor Ti plasmid. This introduction is achieved in Agrobacterium as a host by a single crossover event which occurs within the two homologous DNA segments. The resulting hybrid Ti plasmids in Agrobacterium can be used directly to infect plant cells, that can subsequently be screened for the expression of the product of the gene(s) of interest. From the transformed plant cells it is possible to regenerate transformed fertile plants.

However, the hybrid Ti plasmids of EP-A-0 116 718 that are produced by the single crossover event have two copies of T-DNA subfragments. This duplication is undesirable for the reason referred to above.

A variety of other methods has been reported for inserting DNA into plant cells. One such method involves the use of lipid vesicles, also called liposomes, to encapsulate one or more DNA molecules. The liposomes and their DNA contents may be taken up by plant cells; see, e.g., Lurquin, 1981. If the inserted DNA can be incorporated into the plant genome, replicated, and inherited, the plant cells will be transformed.

Other techniques involve contacting plant cells with DNA which is complexed with either (a) polycationic substances, such as poly-L-ornithine (Davey et al, 1980), or (b) calcium phosphate (Krens et al, 1982). Using these techniques, all or part of a Ti plasmid has been reportedly inserted into plant cells, causing tumorigenic alteration of the plant cells.

Another method has been developed involving the fusion of bacteria, which contain desired plasmids, with plant cells. Such methods involve converting the bacteria into spheroplasts and converting the plant cells into protoplasts. Both of these methods remove the cell wall barrier from the bacterial and plant cells, using enzymic digestion. The two cell types can then be fused together by exposure to chemical agents, such a polyethylene glycol. See Hasezawa et al, 1981.

However, all of the foregoing techniques suffer from one or more of the following problems: (i) transformation efficiencies reported to date have been very low; (ii) only small DNA molecules can be inserted into plant cells; (iii) only small numbers of DNA molecules can be inserted into plant cells; and/or (iv) a gene which is inserted into a plant cell will not be stably maintained by the plant cell unless it is incorporated into the genome of the plant cell, i.e., unless the gene is inserted into a chromosome or plasmid that replicates in the plant cell.

Prior to this invention, no satisfactory method existed for the creation and identification of genetically transformed plant cells which could be routinely regenerated into morphologically normal plants.

SUMMARY OF THE INVENTION

This invention relates to several plasmids which are useful for creating transformed plant cells which are capable of subsequent regeneration into differentiated, morphologically-normal plants. This invention also relates to microorganisms containing such plasmids, and to methods for creating such plasmids and microorganisms.

This invention involves a first plasmid, such as pMON120, which has certain desired characteristics described below. A gene which is capable of being expressed in plant cells may be inserted into this plasmid to obtain a derivative plasmid, such as pMON128. For example, plasmid pMON128 contains a chimeric gene which expresses neomycin phosphotransferase II (NPT II), an enzyme which inactivates certain antibiotics. The chimeric gene is capable of expression in plant cells.

The derivative plasmid is inserted into a suitable microorganism, such as Agrobacterium tumefaciens cells which contain Ti plasmids. In the A. tumefaciens cells, some of the inserted plasmids recombine with Ti plasmids to form a co-integrate plasmid; this is due to a region of homology between the two plasmids. Only a single crossover event is required to create the desired co-integrate plasmid.

Because of the characteristics of the inserted plasmid of this invention, the resulting co-integrate Ti plasmid contains the chimeric gene and/or any other inserted gene within the T-DNA region of the co-integrate plasmid. The inserted gene(s) are surrounded by at least two T-DNA borders, at least one of which was inserted into the Ti plasmid by the crossover event. By means of appropriate antibiotics, A. tumefaciens cells which do not have co-integrate Ti plasmids with inserted genes are killed.

A. tumefaciens cells with co-integrate plasmids are co-cultured with plant cells, such as protoplasts, protoplast-derived cells, plant cuttings, or intact plants, under conditions which allow the co-integrate Ti plasmids, or portions thereof, to enter the plant cells. Once inside the plant cells, a portion of the Ti plasmid which is surrounded by the two T-DNA borders is inserted by natural processes into the plant genome. This segment of DNA contains the chimeric gene and/or any other desired gene(s). Preferably, the segment of vector DNA which is inserted into the plant genome does not contain any genes which would render the plant cell incapable of being regenerated into a differentiated, morphologically-normal plant. The transformed plant cell(s) may be regenerated into a morphologically-normal plant which will pass the inserted gene to its descendants.

A variety of uses exist for plants transformed by the method of this invention. For example, a gene which codes for an enzyme which inactivates a herbicide may be inserted into a plant. This would make the plant and its descendants resistant to the herbicide. Alternately, a gene which codes for a desired mammalian polypeptide such as growth hormone, insulin, interferon, or somatostatin may be inserted into plants. The plants may be grown and harvested, and the polypeptide could be extracted from the plant tissue.

## BRIEF DESCRIPTION OF THE DRAWINGS

FIG. 1 is a flow chart indicating the steps of this invention, using pMON128 and the NOS-NPT II gene as an example.

FIG. 2 represents the creation of pMON41, a plasmid used to construct pMON120.

FIG. 3 represents the creation of M-4, an M13-derived DNA used to construct pMON109.

FIG. 4 represents the creation of pMON54, a plasmid used to construct pMON109.

FIG. 5 represents the creation of pMON109, a plasmid used to construct pMON120.

FIG. 6 represents the creation of pMON113, a plasmid used to construct pMON120.

FIG. 7 represents the creation of plasmid pMON120, an intermediate vector with three restriction endonuclease cleavage sites which are suitable for the insertion of a desired gene.

FIG. 8 represents the creation of pMON128, an intermediate vector which was obtained by inserting a chimeric NOS-NPT II kanamycin-resistance gene into pMON120.

FIG. 9 represents the cointegration of pMON128 with a wild-type Ti plasmid by means of a single crossover event, thereby creating a co-integrate plasmid with multiple borders.

FIG. 10 indicates the co-integration of pMON128 with a disarmed Ti-plasmid, thereby creating a non-tumorigenic cointegrate plasmid.

FIG. 11 is a graph comparing growth of transformed cells and non-transformed cells on kanamycin-containing medium.

## DETAILED DESCRIPTION OF THE INVENTION

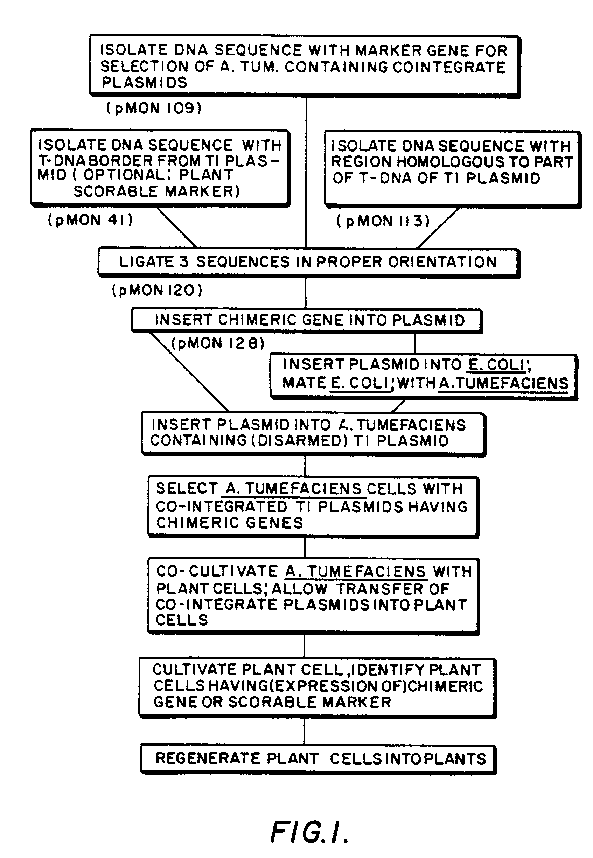

In one preferred embodiment of this invention, a variety of chimeric genes were inserted into plant cells using the steps that are summarized on the flow chart of Figure 1. As shown on Figure 1, three preliminary plasmids were prepared. Those plasmids were designated as:

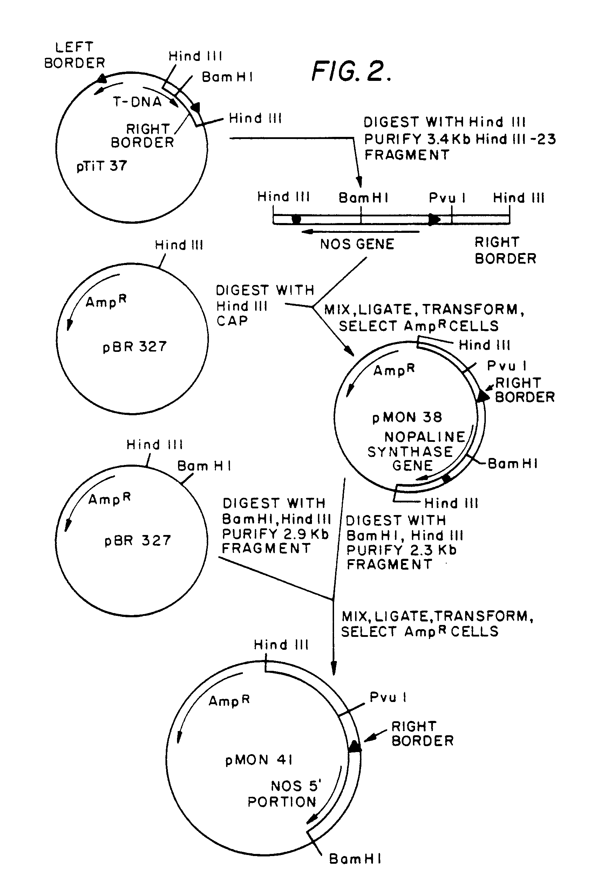

1. pMON41, which contained a right border from a nopaline-type Ti plasmid, and the 5' portion of a nopaline synthase (NOS) gene. The construction of plasmid pMON41 is described below and shown in Figure 2.

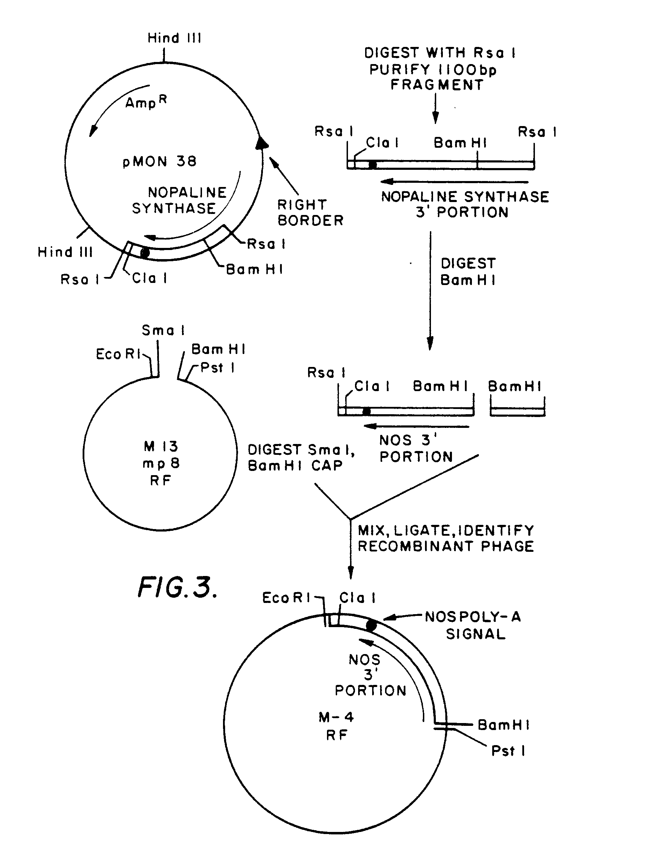

2. pMON109, which contained the 3' portion of a NOS gene, and a selectable marker gene (spc/str) which allowed for the selection of A. tumefaciens cells having co-integrate Ti plasmids with chimeric genes. The construction of plasmid pMON109 is described below and shown in Figures 3, 4, and 5.

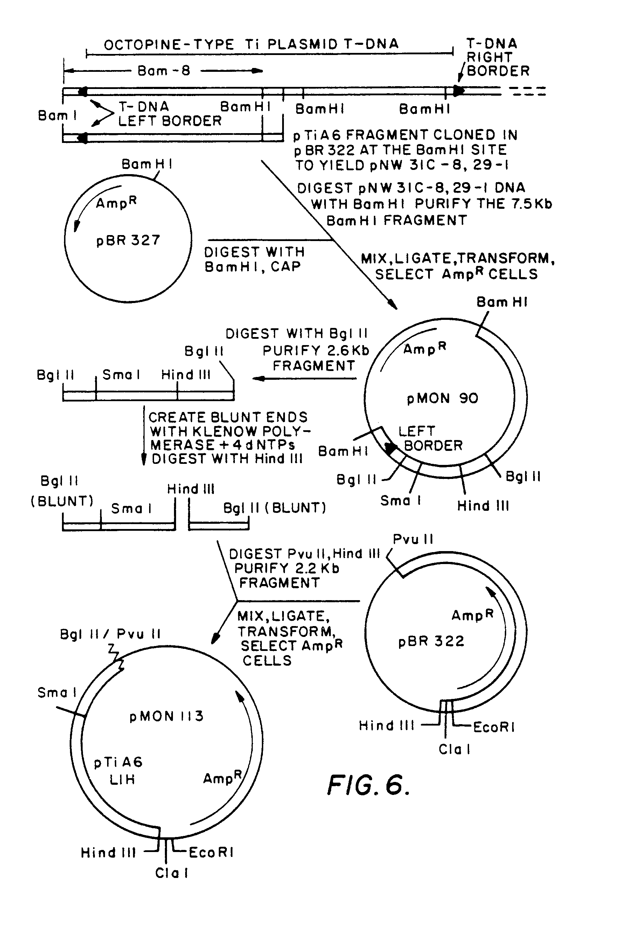

3. pMON113, which contained a region of DNA with a sequence that is identical to the sequence within the T-DNA portion of an octopine-type Ti plasmid. This region was designated as the "left inside homology" (LIH) region. The construction of pMON113 is described below and shown in Figure 6.

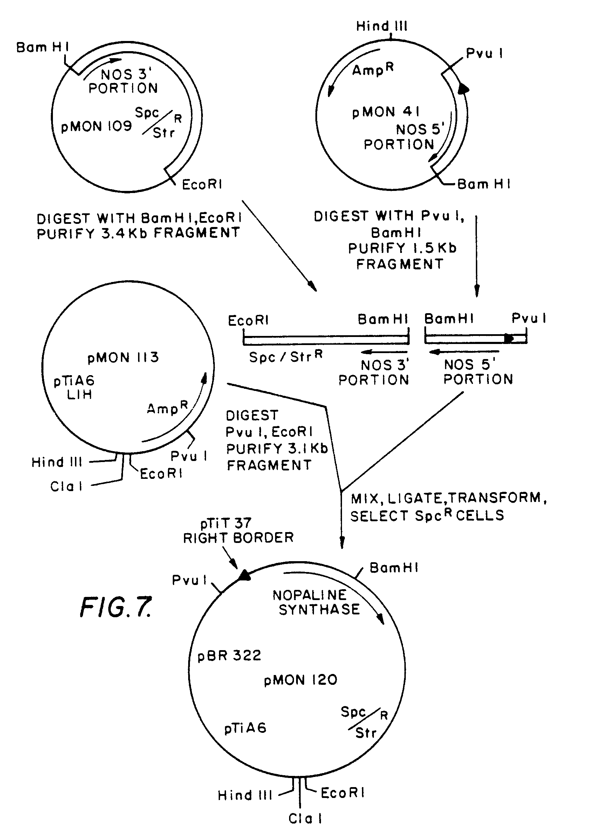

After these plasmids were assembled, each plasmid was digested by appropriate endonucleases to obtain a desired fragment. Three fragments (one from each of the three plasmids) were assembled in a triple ligation to obtain the intermediate vector, plasmid pMON120, as shown in Figure 7.

Plasmid pMON120 plays a key role in the embodiment of this invention that is described in detail below. This plasmid has the following characteristics:

1. pMON120 has at least three unique restriction endonuclease cleavage sites (EcoRI, ClaI, and HindIII) which allow for the convenient insertion of any desired gene.

2. pMON120 will replicate within normal E. coli cells. However, it will not replicate within normal Agrobacterium cells unless it co-integrates with another plasmid, such as a Ti plasmid, which will replicate in Agrobacterium cells.

3. pMON120 carries a marker gene which codes for an enzyme which confers resistance to two antibiotics, spectinomycin (spc) and streptomycin (str). This gene, referred to as the spc/str gene, is expressed in E. coli and in A. tumefaciens, but not in plant cells. pMON120 does not carry genes which code for resistance to ampicillin or tetracycline.

4. pMON120 carries a sequence which is homologous to a sequence within the T-DNA portion of an octopine-type Ti plasmid of A. tumefaciens. This sequence is referred to as the "left inside homology" (LIH) region. This region of homology promotes a crossover event whereby pMON120, or a derivative of pMON120 such as pMON128, forms a co-integrate with the Ti plasmid if the two plasmids exist inside the same A. tumefaciens cell. By definition, the "co-integrate" plasmid is formed by a single crossover event. It contains all DNA sequences that previously existed in either the Ti plasmid or the pMON120-derived plasmid.

5. pMON120 carries a nopaline-type T-DNA "right border," i.e., a sequence which is capable of acting as one end (designated by convention as the right border) of a T-DNA sequence which is transferred from a Ti plasmid and inserted into the chromosorne of a plant cell during transformation of the cell by A. tumefaciens.

6. pMON120 carries a gene (including a promoter) which codes for the expression of an enzyme, nopaline synthase (NOS). Once introduced into a plant cell, the NOS enzyme catalyzes the production of nopaline, a type of opine. In most types of plants, opines are non-detrimental compounds which accumulate at low levels; the presence of nopaline can be readily detected in plant tissue (Otten and Schilperoort, 1978). Opine genes may serve as useful marker genes to confirm transformation, since opines do not normally exist in untransformed plant cells. If desired, the NOS gene in pMON120 may be rendered non-functional by a variety of techniques known to those skilled in the art. For example, a BamHI cleavage site exists within the coding portion of the NOS gene; a stop codon or other appropriate oligonucleotide sequence could be inserted into this site to prevent the translation of NOS.

7. The relative location of the various genes, cleavage sites, and other sequences in pMON120 is very important to the performance of this invention. The entire pMON120 plasmid, or its derivative plasmid such as pMON128, will be contained in the co-integrate Ti plasmid. However, only part of the co-integrate Ti plasmid (the modified T-DNA region) will be inserted into the plant genome. Therefore, only a part of the pMON120-derived plasmid will be inserted into the plant genome. This portion begins at the T-DNA border, and stretches in one direction only to the region of homology. In pMON120, the NOS scorable marker, the spc/str selectable marker, and the three insertion sites are within the portion of pMON120 that would be transferred into the plant genome. However, the pBR322-derived region next to the LIH, and the PvuI cleavage site, probably would not be transferred into the plant genome. Importantly, this arrangement of pMON120 and its derivatives prevents the transfer of more than one region of homology into the plant genome, as discussed below.

8. pMON120 is about 8 kilobases long. This is sufficiently small to allow it to accomplish all of the objectives of this invention. However, if desired, it may be made somewhat smaller by the deletion of one or more nucleotide sequences which are not essential, using methods which are known to those skilled in the art. Such a reduction in size might improve the efficiency or other characteristics of the plasmid when used for this invention or for other purposes, as may be determined by those skilled in the art.

It is recognized that a wide variety of intermediate vectors which differ from pMON120 in one or more respects may be prepared and utilized by those skilled in the art. For example, the NOS marker used for scoring transformed plant cells might be deleted, or replaced or supplemented by a different scorable or selectable marker. One such marker gene might comprise an antibiotic- resistance gene such as the NOS-NPT II-NOS chimeric gene described below. As another example, the spc/str marker gene used for selecting A. tumefaciens cells with co-integrate plasmids might be deleted, or replaced or supplemented by a different scorable or selectable marker that is expressed in Agrobacteria. As another example, a variety of T-DNA borders (such as a nopaline-type "left" border, or an octopine type left or right border) might be utilized. Similarly, more than one border (such as two or more nopaline right borders, or one nopaline right border and one octopine right border) might be inserted into the intermediate vector, in the desired orientation; this may increase the frequency of insertion of the T-DNA into the plant genome, as may be determined by those skilled in the art. It is also possible to insert both left and right borders (of any type) into an intermediate vector. It is also possible to increase the length of the region of homology; this is likely to increase the frequency of the desired single crossover event (Leemans et al, 1981). It is also possible to select an appropriate region of homology from any type of desired plasmid, such as a nopaline or agropine Ti plasmid or an Ri plasmid; such regions will allow the intermediate vector to form a co-integrate with any desired plasmid.

Method of Creating pMON120

Plasmid pMON120 was constructed from fragments derived from 3 other plasmids. These three plasmids were designated as pMON41, pMON109, and pMON113. The construction of each of these three plasmids is summarized below; additional information is provided in the examples.

Plasmid pMON41 contributed a nopaline-type T-DNA right border and the 5' portion of a nopaline synthase (NOS) gene to pMON120. It was created by the following method.

A nopaline-type Ti plasmid, designated as the pTiT37 plasmid, may be digested with the HindIII endonuclease to produce a variety of fragments, including a 3.4 kb fragment which is designated as the HindIII-23 fragment. This fragment contains the entire NOS gene and the T-DNA right border. The Applicants inserted a HindIII-23 fragment into a plasmid, pBR327 (Soberon et al, 1980), which had been digested with HindIII. The resulting plasmid, designated as pMON38, was digested with both HindIII and BamHI. This produced a 2.3 kb fragment which contains the nopaline-type right border and the 5' portion of a NOS gene (including the promoter region, the 5' non-translated region, and part of the structural sequence). This 2.3 kb fragment was inserted into a pBR327 plasmid which had been digested with HindIII and BamHI. The resulting plasmid was designated as pMON41, as shown in Figure 2.

A variety of strains of A. tumefaciens are publicly available from the American Type Culture Collection (Rockville, MD); accession numbers are listed in any ATCC catalog. Each strain contains a Ti plasmid which is likely to be suitable for use in this invention, as may be determined through routine experimentation by those skilled in the art.

Plasmid pMON109 contributed a spc/str selectable marker gene and the 3' portion of a NOS gene to pMON120. It was created by the following method.

Plasmid pMON38 (described above and shown on Figure 2) was digested with RsaI, which creates blunt ends as shown: 5'-GTAC
CATG

A 1.1 kb fragment was isolated, and digested with BamHI to obtain fragments of 720 bp and 400 bp, each of which had one blunt Rsa end and a cohesive BamHI end. These fragments were added to double stranded DNA from a phage M13 mp8 (Messing and Vieira, 1982) which had been digested with SmaI (which creates blunt ends) and BamHI. The mixture was ligated, transformed into cells and plated for recombinant phage. Recombinant phage DNA's which contained the inserted 720 bp fragment were identified by the size of the BamHI-SmaI insert. One of those phage DNA's was designated as M-4, as shown in Figure 3. The 720 bp insert contained the 3' non-translated region (including the poly-adenylation signal indicated in the figures by a heavy dot) of the NOS gene, as well as the 3' portion of the structural sequence of the NOS gene. The 720 bp insert is surrounded in M-4 by EcoRI and PstI cleavage sites, which were present in the M13 mp8 DNA.

A bacterial transposon, designated as Tn7, is known to contain the spc/str gene, mentioned previously. The Tn7 transposon also contains a gene which causes the host cell to be resistant to the antibiotic trimethoprim. The exact location and orientation of the spc/str gene and the trimethoprim-resistance gene in Tn7, are not known. The Tn7 transposon may be obtained from a variety of cell strains which are publicly available. A strain of A. tumefaciens was isolated in which the Tn7 transposon had been inserted into the Hind III-23 region of a pTiT37 plasmid. The modified pTiT37 plasmid was designated as pGV3106 (Hernalsteens et al, 1980).

Plasmid pGV3106 was digested with HindIII, and the fragments were shotgun-cloned into pBR327 plasmids which had been digested with HindIII. These plasmids were inserted into E. coli cells, and cells which were ampicillin-resistant (due to a pBR327 gene) and trimethoprim-resistant (due to a Tn7 gene) were selected. The plasmid obtained from one colony was designated as pMON31. This plasmid contained a 6kb HindIII insert. The insert contained the spc/str-resistance gene and trimethoprim-resistance gene from Tn7, and the 3' portion of a NOS gene (which came from the pTiT37 plasmid).

Plasmid pMON31 was reduced in size twice. The first reduction was performed by digesting the plasmid with EcoRI, diluting the mixture to remove an 850 bp fragment, and religating the large fragment. The resulting plasmid, designated as pMON53, was obtained from transformed cells selected by their resistance to ampicillin and streptomycin. Resistance to trimethoprim was not determined.

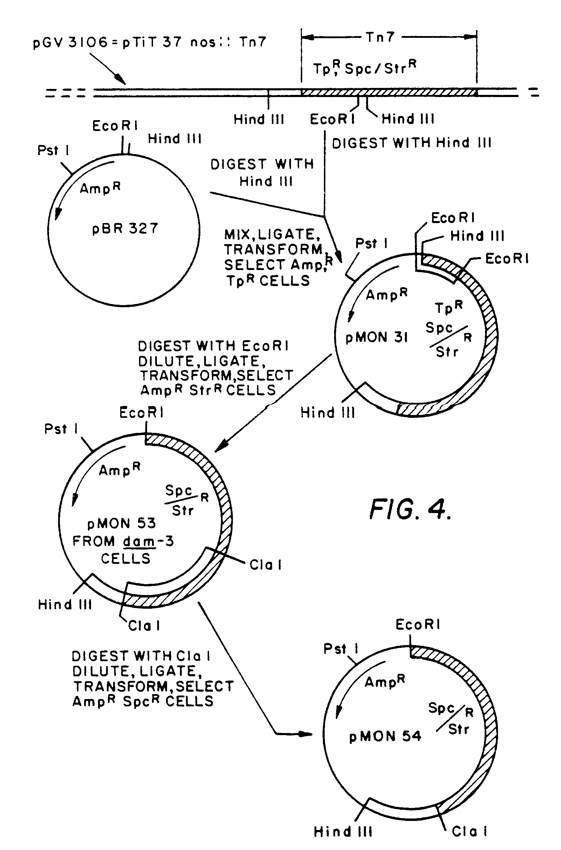

Plasmid pMON53 was further reduced in size by digesting the plasmid with ClaI, diluting the mixture to remove a 2 kb fragment, and religating the large fragment. The resulting 5.2 kb plasmid was designated as pMON54, as shown in Figure 4. This plasmid contains the spc/str gene.

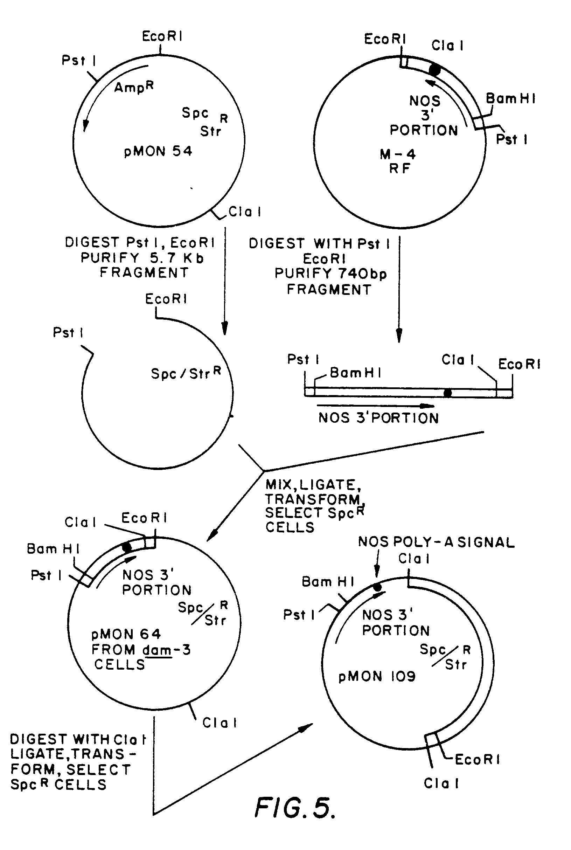

Plasmid pMON54 was digested with EcoRI and PstI, and a 4.8 kb fragment containing the spc/str gene was isolated. M-4 DNA was digested with EcoRI and PstI, and a 740 bp fragment containing the NOS 3' non-translated region was isolated. These fragments were ligated together to form pMON64. In order to be able to obtain the NOS 3' portion and the spc/str gene on a single EcoRI-BamHI fragment, the orientation of the spc/str gene was reversed by digesting pMON64 with ClaI and religating the mixture. Plasmids having the desired orientation were identified by cleavage using EcoRI and BamHI. These plasmids were designated as pMON109, as shown in Figure 5.

Plasmid pMON113 contributed a region of homology to pMON120 which allows pMON120 to form a co-integrate plasmid when present in A. tumefaciens along with a Ti plasmid. The region of homology was taken from an octopine-type Ti plasmid. In the Ti plasmid, it is located near the left T-DNA border, within the T-DNA portion of the Ti plasmid. This region of homology is designated as the "left inside homology" (LIH) region.

A region of homology may be derived from any type of plasmid capable of transforming plant cells, such as any Ti plasmid or any Ri plasmid. An intermediate vector can be designed which can form a co-integrate plasmid with whatever type of plasmid the region of homology was derived from.

In addition, it might not be necessary for the region of homology to be located within the T-DNA. For example, it may be possible for a region of homology to be derived from a segment of a Ti plasmid which contains a T-DNA border and a sequences of bases outside of the T-DNA region. Indeed, if the intermediate vector contains two appropriate T-DNA borders, it might be possible for the region of homology to be located entirely outside of the T-DNA region.

The Applicants obtained an E. coli culture with a pBR-derived plasmid containing the Bam-8 fragment of an octopine-type Ti plasmid. The Bam-8 fragment, which is about 7.5 kb, contains the left border and the LIH region of the Ti plasmid (Willmitzer et al, 1982; DeGreve et al, 1981). The Bam-8 fragment was inserted into the plasmid pBR327, which had been digested with BamHI. The resulting plasmid was designated as pMON90, as shown in Figure 6.

Plasmid pMON90 was digested with BglII, and a 2.6 kb fragment which contains the LIH region but not the left border was purified. The 2.6 kb fragment was treated with Klenow polymerase to convert the cohesive ends into blunt ends, and the fragment was digested with HindIII to obtain a 1.6 kb fragment (the desired fragment) and a 1 kb fragment. Both fragments were mixed with a pBR322 plasmid which had been digested with PvuII and HindIII. The mixture was ligated, and inserted into E. coli cells. The cells were selected for ampicillin resistance, and scored for the presence of a SmaI site which exists on the 1.6 kb fragment but not the 1 kb fragment. A colony having the desired plasmid was identified, and the plasmid from this colony was designated as pMON113, as indicated by Figure 6.

To assemble pMON120, three fragments had to be isolated. Plasmid pMON41 was digested with PvuI and BamI, and a 1.5 kb fragment containing a nopaline-type right border and the 5' portion of a NOS gene was isolated. Plasmid pMON109 was digested with BamHI and EcoRI, and a 3.4 kb fragment containing a spc/str gene and the 3' part of a NOS gene was isolated. Plasmid pMON113 was digested with PvuI and EcoRI, and a 3.1 kb fragment containing the LIH region was isolated.

The three fragments were mixed together and ligated to form pMON120, as shown on Figure 7. A culture of E. coli containing pMON120 has been deposited with the American Type Culture Collection. This culture has been assigned accession number 39263.

It is recognized that a variety of different methods could be used to create pMON120, or any similar intermediate vector. For example, instead of the triple ligation, it would have been possible to assemble two of the desired fragments in a plasmid, and insert the third fragment into the plasmid.

Method of Using pMON120

As mentioned previously, pMON120 has three unique cleavage sites (EcoRI, ClaI, and HindIII) which are suitable for the insertion of any desired gene. These cleavage sites are located in the portion of pMON120 that will be inserted into a plant genome, so the inserted gene also will be inserted into the plant genome.

A variety of chimeric genes which are capable of expressing bacterial and mammalian polypeptides in plant cells have been created by the Applicants. These chimeric genes are described in detail in a separate application entitled "Chimeric Genes Suitable for Expression in Plant Cells," copending U.S. application serial number 458,414, filed on January 17, 1983.

Those chimeric genes are suitable for use in this invention. They may be inserted into pMON120 to create a derivative plasmid, which may be utilized as described below.

In one preferred embodiment of this invention, a chimeric gene was created which comprises the following DNA sequences:

1. a promoter region and a 5' non-translated region derived from a nopaline synthase (NOS) gene;

2. a structural sequence derived from a neomycin phosphotransferase II (NPT II) gene; and

3. a 3' non-translated region derived from a NOS gene.

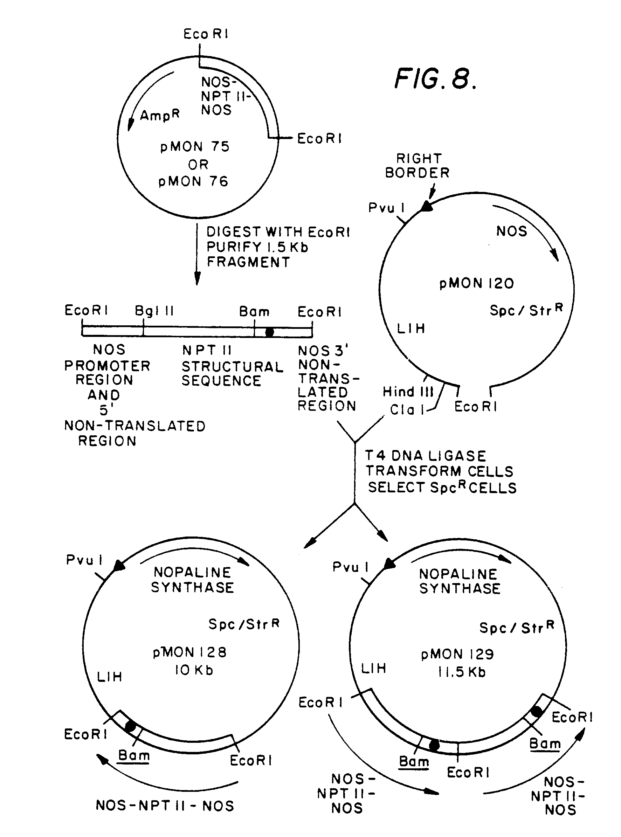

This chimeric NOS-NPT II-NOS gene was isolated on a DNA fragment having EcoRI ends. This fragment was inserted into the EcoRI cleavage site of pMON120, and the resulting plasmids (having chimeric gene inserts with opposite orientations) were designated as pMON128 and pMON129, as shown in Figure 8. Plasmid pMON129 has two copies of the chimeric gene; this may be a useful feature in certain types of work. Either plasmid may be utilized to transform plant cells, in the following manner. A culture of E. coli containing pMON128 has been deposited with the American Type Culture Collection. This culture has been assigned accession number 39264.

Plasmid pMON128 (or any other plasmid derived by inserting a desired gene into pMON120) is inserted into a microorganism which contains an octopine-type Ti plasmid (or other suitable plasmid). Suitable microorganisms include A. tumefaciens and A. rhizogenes which carry Ti or Ri plasmids. Other microorganisms which might also be useful for use in this invention include other species of Agrobacterium, as well as bacteria in the genus Rhizobia. The suitability of these cells, or of any other cells known at present or hereafter discovered or created, for use in this invention may be determined through routine experimentation by those skilled in the art.

The plasmid may be inserted into the microorganism by any desired method, such as transformation (i.e., contacting plasmids with cells that have been treated to increase their uptake of DNA) or conjugation with cells that contain the pMON128 or other plasmids.

The inserted plasmid (such as pMON128) has a region which is homologous to a sequence within the Ti plasmid. This "LIH" region of homology allows a single crossover event whereby pMON128 and an octopine-type Ti plasmid combine with each other to form a co-integrate plasmid. See, e.g., Stryer, supra, at p. 752-754. Normally, this will occur within the A. tumefaciens cell after pMON128 has been inserted into the cell. Alternately, the co-integrate plasmid may be created in a different type of cell or in vitro, and then inserted into an A. tumefaciens or other type of cell which can transfer the co-integrate plasmid into plant cells.

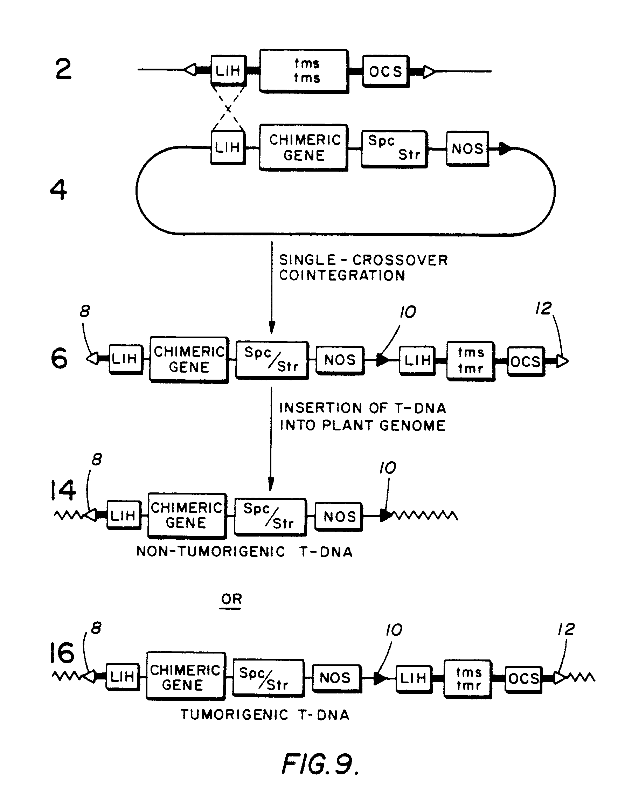

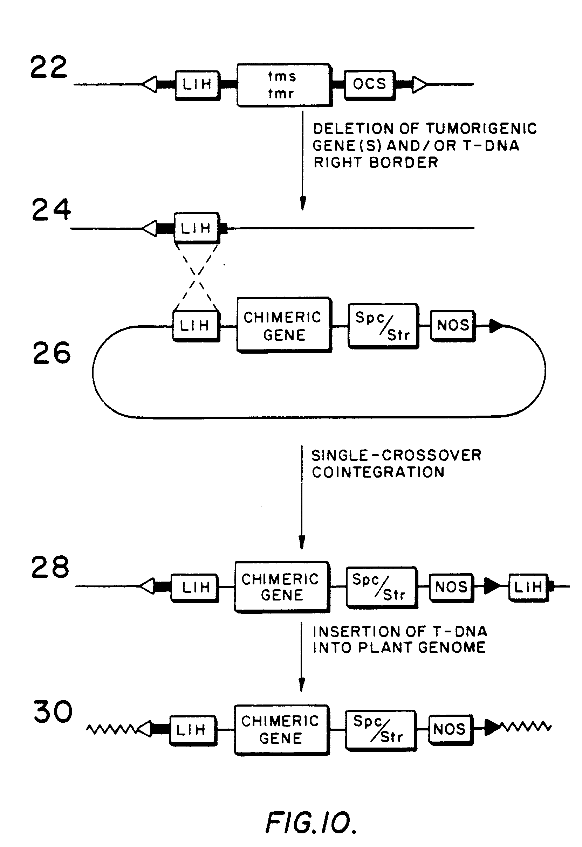

The inserted plasmid, such as pMON128, combines with the Ti plasmid in the manner represented by Figures 9 or 10, depending upon what type of Ti plasmid is involved.

In Figure 9, item 2 represents the T-DNA portion of an octopine-type Ti plasmid. Item 4 represents the inserted plasmid, such as pMON128. When these two plasmids co-exist in the same cell, a crossover event can occur which results in the creation of co-integrate plasmid 6.

Co-integrate plasmid 6 has one left border 8, and two right borders 10 and 12. The two right borders are designated herein as the "proximal" right border 10 (the right border closest to left border 8), and the "distal" right border 12 (the right border that is more distant from left border 8. Proximate right border 10 was carried by plasmid 4; the distal right border was contained on Ti plasmid 2 before co-integration.

A culture of A. tumefaciens GV3111 containing a co-integrate plasmid formed by pMON128 and wild-type Ti plasmid pTiB653 has been deposited with the American Type Culture Center. This culture has been assigned accession number 39266.

When co-integrate Ti plasmid 6, shown in Figure 9, is inserted into a plant cell, either of two regions of DNA may enter the plant genome, T-DNA region 14 or T-DNA region 16.

T-DNA region 14 is bounded by left border 8 and proximate right border 10. Region 14 contains the chimeric gene and any other genes contained in plasmid 4, such as the spc/str selectable marker and the NOS scorable marker. However, region 14 does not contain any of the T-DNA genes which would cause crown gall disease or otherwise disrupt the metabolism or regenerative capacity of the plant cell.

T-DNA region 16 contains left border 8 and both right borders 10 and 12. This segment of T-DNA contains the chimeric gene and any other genes contained in plasmid 4. However, T-DNA region 16 also contains the T-DNA genes which are believed to cause crown gall disease.

Either of the foregoing T-DNA segments, Region 14 or Region 16, might be transferred to the plant DNA. This is presumed to occur at a 50-50 probability for any given T-DNA transfer. This is likely to lead to a mixture of transformed cells, some of which are tumorous and some or which are non-tumorous. It is possible to isolate and cultivate non-tumorous cells from the mixture, as described in the examples.

An alternate approach has also been developed which avoids the need for isolating tumorous from non-tumorous cells. Several mutant strains of A.tumefaciens have been isolated which are incapable of causing crown gall disease. Such strains are usually referred to as "disarmed" Ti plasmids. A Ti or Ri plasmid may be disarmed by one or more of the following types of mutations:

1. Removal or inactivation of one of the border regions. One such disarmed octopine plasmid, which has a left border but not a right border, is designated as pAL4421; this plasmid is contained in A. tumefaciens strain LBA4421 (Ooms et al, 1982; Garfinkel et al, 1981).

2. Removal or inactivation of the one or more of the "tumor morphology" genes, designated as the tmr and tms genes. See, e.g., Leemans et al, 1982.

Various other types of disarmed Ti plasmids may be prepared using methods known to those skilled in the art. See Matzke and Chilton, 1981; Leemans et al, 1981; Koekman et al, 1979.

Figure 10 represents an octopine-type Ti plasmid with a T-DNA region 22 which undergoes mutation to delete the tms and tmr genes and the right border. This results in a disarmed Ti plasmid with partial T-DNA region 24. When plasmid 26 (such as pMON128) is inserted into a cell that carries the disarmed Ti plasmid 24, a crossover event occurs which creates a co-integrate Ti plasmid with disarmed T-DNA region 28. The LIH region of homology is repeated in this Ti plasmid, but the disarmed Ti plasmid does not contain any oncogenic genes. Alternately, if only the right border had been deleted from T-DNA region 22, then the tms and tmr genes and the octopine synthase (OCS) gene would be contained in the co-integrate disarmed Ti plasmid; however, they would have been located outside of the T-DNA borders.

The disarmed co-integrate Ti plasmid is used to infect plant cells, and T-DNA region 28 enters the plant genome, as shown by transformed DNA 30. Plant cells transformed by disarmed T-DNA 28 have normal phytohormone metabolism, and normal capability to be regenerated into differentiated plants.

After pMON128 is inserted into A. tumefaciens cells, the desired crossover event will occur in a certain fraction of the cells. Cells which contain co-integrate plasmids (whether virulent or disarmed) may be easily selected from other cells in which the crossover did not occur, in the following manner. Plasmid pMON120 and its derivatives contain a marker gene (spc/str), which is expressed in A. tumefaciens. However, these plasmids do not replicate in A. tumefaciens. Therefore, the spc/str marker gene will not be replicated or stably inherited by A. tumefaciens unless the inserted plasmid combines with another plasmid that can replicate in A. tumefaciens. The most probable such combination, due to the region of homology, is the co-integrate formed with the Ti plasmid. A. tumefaciens cells which contain this co-integrate plasmid can readily be identified and selected by growth of the cells on medium containing either spc or str, or both.

The Ti plasmid 28, shown in Figure 10, contains two LIH regions. It is possible that co-integrate Ti plasmids will undergo a subsequent crossover event, wherein the two LIH regions will recombine. This event is undesirable, since it can lead to a deletion of the DNA between the LIH regions, including the chimeric gene. However, this is not likely to lead to serious difficulties, for two reasons. First, this event is likely to occur at a relatively low probability, such as about 10-2. Second, plasmid pMON120 and its derivatives have been designed so that the selectable marker gene (spc/str) is located in the region of DNA that would be deleted by the crossover event. Therefore, the selective conditions that are used to identify and culture Agrobacteria cells containing co-integrate plasmids will also serve to kill the descendants of cells that undergo a subsequent crossover event which eliminates the chimeric gene from the Ti plasmid.

Only one of the LIH regions in the co-integrate Ti plasmid will be inserted into the plant genome, as shown in Figure 10. This important feature results from the design of pMON120, and it distinguishes this co-integrate plasmid from undesired co-integrate plasmids formed by the prior art. The LIH region which lies outside of the T-DNA borders will not be inserted into the plant genome. This leads to at least two important advantages. First, the presence of two LIH regions inserted into the plant genome could result in crossover events which would lead to loss of the inserted genes in the transformed plant cells and their progeny. Second, the presence of two regions of DNA homology can significantly complicate efforts to analyze the DNA inserted into the plant genome (Matzke and Chilton, 1981).

After A. tumefaciens cells which contain the co-integrate Ti plasmids with the chimeric genes have been identified and isolated, the co-integrate plasmids (or portions thereof) must be inserted into the plant cells. Eventually, methods may be developed to perform this step directly. In the meantime, a method has been developed which may be used conveniently and with good results. This method is described in two separate copending U.S. patent applications, entitled "Transformation of Plant Cells by Extended Bacterial Co-Cultivation" serial number 458,413, and "Rapid Culture of Plant Protoplasts," serial number 458,412, both of which were filed on January 17, 1983. The method described in those applications may be briefly summarized as follows.

The plant cells to be transformed are contacted with enzymes which remove the cell walls. This converts the plant cells into protoplasts, which are viable cells surrounded by membranes. The enzymes are removed, and the protoplasts begin to regenerate cell wall material. At an appropriate time, the A. tumefaciens cells (which contain the co-integrate Ti plasmids with chimeric genes) are mixed with the plant protoplasts. The cells are co-cultivated for a period of time which allows the A. tumefaciens to infect the plant cells. After an appropriate co-cultivation period, the A. tumefaciens cells are killed, and the plant cells are propagated.

Plant cells which have been transformed (i.e., cells which have received DNA from the co-integrate Ti plasmids) and their descendants may be selected by a variety of methods, depending upon the type of gene(s) that were inserted into the plant genome. For example, certain genes may cause various antibiotics to be inactivated; such genes include the chimeric NOS-NPT II-NOS gene carried by pMON128. Such genes may serve as selectable markers; a group of cells may be cultured on medium containing the antibiotic which is inactivated by the chimeric gene product, and only those cells containing the selectable marker gene will survive.

A variety of genes may serve as scorable markers in plant cells. For example, pMON120 and its derivative plasmids, such as pMON128, carry a nopaline synthase (NOS) gene which is expressed in plant cells. This gene codes for an enzyme which catalyzes the formation of nopaline. Nopaline is a non-detrimental compound which usually is accumulated at low quantities in most types of plants; it can be easily detected by electrophoretic or chromatographic methods.

If a plant is transformed by a gene which creates a polypeptide that is difficult to detect, then the presence of a selectable marker gene (such as the NOS-NPT II-NOS chimeric gene) or a scorable marker gene (such as the NOS gene) in the transforming vector may assist in the identification and isolation of transformed cells.

Virtually any desired gene may be inserted into pMON120 or other plasmids which are designed to form co-integrates with Ti or similar plasmids. For example, the Applicants have created a variety of chimeric genes, which are discussed in the previously-cited application, "Chimeric Genes Suitable for Expression in Plant Cells."

The suitability of any gene for use in this invention may be determined through routine experimentation by those skilled in the art. Such usage is not limited to chimeric genes; for example, this invention may be used to insert multiple copies of a natural gene into plant cells.

This invention is suitable for use with a wide variety of plants, as may be determined through routine experimentation by those skilled in the art. For example, this invention is likely to be useful to transform cells from any type of plant which can be infected by bacteria from the genus Agrobacterium. It is believed that virtually all dicotyledonous plants, and certain monocots, can be infected by one or more strains of Agrobacterium. In addition, microorganisms of the genus Rhizobia are likely to be useful for carrying co-integrate plasmids of this invention, as may be determined by those skilled in the art. Such bacteria might be preferred for certain types of transformations or plant types.

Certain types of plant cells can be cultured in vitro and regenerated into differentiated plants using techniques known to those skilled in the art. Such plant types include potatoes, tomatoes, carrots, alfalfa and sunflowers. Research in in vitro plant culture techniques is progressing rapidly, and methods are likely to be developed to permit the regeneration of a much wider range of plants from cells cultured in vitro. Cells from any such plant with regenerative capacity are likely to be transformable by the in vitro co-cultivation method discussed previously, as may be determined through routine experimentation by those skilled in the art. Such transformed plant cells may be regenerated into differentiated plants using the procedures described in the examples.

The in vitro co-cultivation method offers certain advantages in the transformation of plants which are susceptible to in vitro culturing and regeneration. However, this invention is not limited to in vitro cell culture methods. For example, a variety of plant shoots and cuttings (including soybeans, carrots, and sunflowers) have been transformed by contact with A. tumefaciens cells carrying the co-integrate plasmids of this invention. It is also possible to regenerate virtually any type of plant from a cutting or shoot. Therefore, it may be possible to develop methods of transforming shoots or cuttings using virulent or preferably disarmed co-integrate plasmids of this invention or mixtures thereof, and subsequently regenerating the transformed shoots or cuttings into differentiated plants which pass the inserted genes to their progeny.

As mentioned previously, it is not essential to this invention that co-cultivation be utilized to transfer the co-integrate plasmids of this invention into plant cells. A variety of other methods are being used to insert DNA into cells. Such methods include encapsulation of DNA in liposomes, complexing the DNA with chemicals such as polycationic substances or calcium phosphate, fusion of bacterial spheroplasts with plant protoplasts, microinjection of DNA into a cell, and induction of DNA uptake by means of electric current. Although such methods have not been used to insert DNA into plant cells with satisfactory efficiency to date, they are being actively researched and they may be useful for inserting foreign genes into plant cells, using the intermediate vectors and co-integrate plasmids of this invention, or plasmids derived therefrom.

This invention may be useful for a wide variety of purposes. For example, certain bacterial enzymes, such as 5-enol pyruvyl shikimate-3-phosphoric acid synthase (EPSP synthase) are inactivated by certain herbicides; other enzymes, such as glutathione-S-transferase (GST), inactivate certain herbicides. It may be possible to insert chimeric genes into plants which will cause expression of such enzymes in the plants, thereby causing the plants to become resistant to one or more herbicides. This would allow for the herbicide, which would normally kill the untransformed plant, to be applied to a field of transformed plants. The herbicide would serve as a weed-killer, leaving the transformed plants undamaged.

Alternately, it may be possible to insert chimeric genes into plants which will cause the plants to create mammalian polypeptides, such as insulin, interferon, growth hormone, etc. At an appropriate time, the plants (or cultured plant tissue) would be harvested. Using a variety of processes which are known to those skilled in the art, the desired protein may be extracted from the harvested plant tissue.

An alternate use of this invention is to create plants with high content of desired substances, such as storage proteins or other proteins. For example, a plant might contain one or more copies of a gene which codes for a desirable protein. Additional copies of this gene may be inserted into the plant by means of this invention. Alternately, the structural sequence of the gene might be inserted into a chimeric gene under the control of a different promoter which causes prolific transcription of the structural sequence.

The methods of this invention may be used to identify, isolate, and study DNA sequences to determine whether they are capable of promoting or otherwise regulating the expression of genes within plant cells. For example, the DNA from any type of cell can be fragmented, using partial endonuclease digestion or other methods. The DNA fragments can be inserted randomly into plasmids similar to pMON128. These plasmids, instead of having a full chimeric gene such as NOS-NPT II-NOS, will have a partial chimeric gene, with a cleavage site for the insertion of DNA fragments, rather than a NOS promoter or other promoter. The plasmids with inseted DNA are then inserted into A. tumefaciens, where they can recombine with the Ti plasmids. Cells having co-integrate plasmids are selected by means of the spc/str or other marker gene. The co-integrate plasmids are then inserted into the plant cells, by bacterial co-cultivation or other means. The plant cells will contain a selectable marker structural sequence such as the NPT II structural sequence, but this structural sequence will not be transcribed unless the inserted DNA fragment serves as a promoter for the structural sequence. The plant cells may be selected by growing them on medium containing kanamycin or other appropriate antibiotics.

Using this method, it is possible to evaluate the promoter regions of bacteria, yeast, fungus, algae, other microorganisms, and animal cells to determine whether they function as gene promoters in various types of plant cells. It is also possible to evaluate promoters from one type of plant in other types of plant cells. By using similar methods and varying the cleavage site in the starting plasmid, it is possible to evaluate the performance of any DNA sequence as a 5' non-translated region, a 3' non-translated region, or any other type of regulatory sequence.

As used herein, "a piece of DNA" includes plasmids, phages, DNA fragments, and polynucleotides, whether natural or synthetic.

As used herein, a "chimeric piece of DNA" is limited to a piece of DNA which contains at least two portions (i.e., two nucleotide sequences) that were derived from different and distinct pieces of DNA. For example, a chimeric piece of DNA cannot be created by merely deleting one or more portions of a naturally existing plasmid. A chimeric piece of DNA may be produced by a variety of methods, such as ligating two fragments from different plasmids together, or by synthesizing a polynucleotide wherein the sequence of bases was determined by analysis of the base sequences of two different plasmids.

As used herein, a chimeric piece of DNA is limited to DNA which has been assembled, synthesized, or otherwise produced as a result of man-made efforts, and any piece of DNA which is replicated or otherwise derived therefrom. "Man-made efforts" include enzymatic, cellular, and other biological processes, if such processes occur under conditions which are caused, enhanced, or controlled by human effort or intervention; this excludes plasmids, phages, and polynucleotides which are created solely by natural processes. As used herein, the term "derived from" shall be construed broadly. Whenever used in a claim, the term "chimeric" shall be a material limitation.

As used herein, "foreign" DNA includes any DNA which is inserted into a pre-existing plant cell. A "foreign gene" is a gene which is inserted into a pre-existing plant cell.

As used herein, a "marker gene" is a gene which confers a phenotypically identifiable trait upon the host cell which allows transformed host cells to be distinguished from non-transformed cells. This includes screenable, scorable, and selectable markers.

As used herein, a "region of homology" refers to a sequence of bases in one plasmid which has sufficient correlation with a sequence of bases in a different plasmid to cause recombination of the plasmid to occur at a statistically determinable frequency. Preferably, such recombination should occur at a frequency which allows for the convenient selection of cells having combined plasmids, e.g., greater than 1 per $10^6$ cells. This term is described more fully in a variety of publications, e.g., Leemans et al, 1981.

By "tumorigenic genes" is meant those genes of the Ti plasmid whose presence in the transformed plant results in a morphologically abnormal plant, i.e. induces crown gall formation.

Those skilled in the art will recognize, or be able to ascertain using no more than routine experimentation, numerous equivalents to the specific embodiments of the invention discussed herein. Such equivalents are within the scope of this invention.

## Example 1: Creation of Plasmid pMON41

A culture of E. coli, carrying a pBR325 plasmid (Bolivar, 1978) with the HindIII-23 fragment of pTiT37 (Hernalsteens, et al, 1980) inserted at the HindIII site, was obtained from Drs. M. Bevan and M.D. Chilton, Washington University, St. Louis, MO. Ten micrograms (ug) of the plasmid from this clone was digested with 10 units of HindIII (unless noted, all restriction endonucleases used in these constructions were purchased from New England Biolabs, Beverly, MA and used with buffers according to the supplier's instructions) for 1 hour at 37°C. The 3.4 kb HindIII-23 fragment was purified by adsorption on glass beads (Vogelstein and Gillespie, 1979) after separation from the other HindIII fragments by electrophoresis on a 0.8% agarose gel. The purified 3.4 kb HindIII fragment (1.0 ug) was mixed with 1.0 ug of plasmid pBR327 DNA (Soberon, et al, 1980) that had been digested with both HindIII (2 units, 1 hour, 37°C) and calf alkaline phosphatase (CAP; 0.2 units, 1 hour, 37°C), de-proteinized with phenol, ethanol precipitated, and resuspended in 10 ul of TE (10 mM Tris HCl, pH8, 1 mM EDTA). One unit of T4 DNA ligase (prepared by the method of Murray et al, 1979) was added to the fragment mixture. One unit is defined as the concentration sufficient to obtain greater than 90% circularization of one microgram of HindIII linearized pBR327 plasmid in 5 minutes at 22°C. The mixed fragments were contained in a total volume of 15 ul of 25 mM Tris-HCl pH8, 10 mM $MgCl_2$, 1 mM dithiothreitol, 200 uM spermidine HCl and 0.75 mM ATP (ligase buffer).

The mixture was incubated at 22°C for 3 hours and then mixed with E. coli C600 recA56 cells that were prepared for transformation by treatment with $CaCl_2$ (Maniatis et al, 1982). Following a period for expression of the ampicillin resistance determinant carried by the pBR327 vector, cells were spread on LB solid medium plates (Miller, 1972) containing ampicillin at 200 ug/ml. After incubation at 37°C for 16 hours, several hundred clones were obtained. Plasmid mini-preps (Ish-Horowicz and Burke, 1981) were performed on 24 of these colonies and aliquots of the plasmid DNA's obtained (0.1 ug) were digested with HindIII to demonstrate the presence of the 3.4 kb HindIII fragment. One plasmid demonstrated the expected structure and was designated pMON38. pMON38 DNA were prepared by Triton X-100 lysis and CsCl gradient procedure (Davis et al, 1980).

Fifty ug of pMON38 DNA were digested with HindIII and BamHI (50 units each, 2 hours, 37°C)and the 2.3 kb HindIII-BamHI fragment was purified as described above. The purified fragment (1 ug) was mixed with 1 ug of the 2.9 kb HindIII-BamHI fragment of the pBR327 vector purified as described above. Following ligation (T4 DNA ligase, 2 units) and transformation of E. coli cells as described above, fifty ampicillin-resistant colonies were obtained. DNAs from twelve plasmid mini-preps were digested with HindIII and BamHI to ascertain the presence of the 2.3 kb fragment. One plasmid of the correct structure was chosen and designated pMON41, as shown in Figure 2. A quantity of this DNA was prepared as described above.

## Example 2: Creation of M13 Clone M-4

Thirty ug of plasmid pMON38 (described in Example 1) were digested with RsaI (30 units, 2 hours, 37°C) and the 1100 bp RsaI fragment was purified after separation by agarose gel electrophoresis using the glass bead method described in the previous example. The purified 1100 bp RsaI-RsaI fragment (1 ug) was digested with 2 units of BamHI and the BamHI was inactivated by heating. This DNA was mixed with 0.2 ug of phage M13mp8RF DNA which had been previously digested with SmaI and BamHI (2 units each, 1 hour, 37°C) and 0.2 units of calf alkaline phosphotase (CAP). Following ligation with 100 units of T4 DNA ligase, transformation of E. coli JM101 cells as described in the previous example, the transformed cells were mixed with soft agar and plated under conditions that allow the identification of recombinant phage (Messing and Vieira, 1982). Twelve recombinant phage producing cells were picked and RF plasmid mini-preps were obtained as described in the previous example. The RF DNAs were digested with BamHI and SmaI to prove the presence of the 720 bp RsaI-BamHI fragment. One of the recombinant RF DNAs carrying the correct fragment was designated M13 mp8 M-4. This procedure is represented in Figure 3. M-4 RF DNA was prepared using the procedures of Ish-Horowicz and Burke, 1981 and Colman et al, 1978.

## Example 3: Construction of pMON109

Twenty ug of plasmid pGV3106 (Hernalsteens et al 1980, prepared by the method of Currier and Nester 1976) was digested with HindIII (20 units, 2 hours, 37°C) and mixed with 2 ug of HindIII-digested pBR327. Following ligation (T4 DNA ligase, 2 units) and transformation of E. coli cells as described above, one colony resistant to trimethoprim (100 ug/ml) and ampicillin was obtained. Digestion of plasmid DNA from this cell demonstrated the presence of a 6 kb HindIII fragment. This plasmid was designated pMON31.

Plasmid pMON31 from a mini-prep (0.5 ug) was digested with EcoRI (1 unit, 1 hour, 37°C) and the endonuclease was inactivated by heating (10 min, 70°C). The 8.5 kb plasmid fragment was re-circularized in a ligation reaction of 100 ul (T4 DNA ligase, 1 unit) and used to transform E. coli cells with selection for ampicillin and streptomycin (25 ug/ml) resistant colonies. Plasmid mini-prep DNA's from six clones were digested with EcoRI to ascertain loss of the 850 bp fragment. One plasmid lacking the 850 bp EcoRI fragment was designated pMON53. This plasmid was introduced into E. coli GM42 dam- cells (Bale et al, 1979) by transformation as described.

Plasmid pMON53 (0.5 ug) from a mini-prep prepared from dam- cells was digested with ClaI, and recircularized in dilute solution as described above. Following transformation of E. coli GM42 cells and selection for ampicillin and spectinomycin (50 ug/ml) resistant clones, fifty colonies were obtained. Digestion of plasmid mini-prep DNA's from six colonies showed that all lacked the 2 kb ClaI fragment. One of these plasmids was designated pMON54, as represented in Figure 4. Plasmid DNA was prepared as described in Example 1.

Plasmid pMON54 DNA (20 ug) was digested with EcoRI and PstI (20 units of each, 2 hours, 37°C) and the 4.8 kb fragment was purified from agarose gels using NA-45 membrane (Schleicher and Schuell, Keene, N.H.).

The purified 4.8 kb fragment (0.5 ug) was mixed with 0.3 ug of a 740 bp EcoRI-PstI fragment obtained from M13mp8 M-4 RF DNA (described in Example 2) which was purified using NA-45 membrane. Following ligation (T4 DNA ligase, 2 units), transformation of E. coli GM42 dam- cells, and selection for spectinomycin resistant cells, twenty colonies were obtained. Plasmid mini-prep DNA's prepared from twelve of these clones were digested with PstI and EcoRI to demonstrate the presence of the 740 bp fragment. One plasmid carrying this fragment was designated pMON64. A quantity of this plasmid DNA was prepared as described in Example 1.

DNA (0.5 ug) of pMON64 was digested with ClaI (1 unit, 1 hour, 37°C), the ClaI was heat inactivated, and the fragments rejoined with T4 DNA ligase ( 1 unit). Following transformation and selection for spectinomycin resistant cells, plasmid mini-preps from twelve colonies were made. The DNA's were digested with BamHI and EcoRI to determine the orientation of the 2 kb ClaI fragment. Half of the clones contained the ClaI fragment in the inverse orientation of that in pMON64. One of these plasmids was designated pMON109, as represented in Figure 5. DNA was prepared as described in Example 1.

## Example 4: Creation of plasmid pMON113

Plasmid pNW31C-8,29C (Thomashow et al, 1980) was obtained from Dr. S. Gelvin of Purdue University, West Lafayette, IN. This plasmid carries the pTiA6 7.5 kb Bam-8 fragment. The Bam-8 fragment was purified from 50 ug of BamHI-digested pNW31C-8,29C using NA-45 membrane as described in previous examples. The purified 7.5 kb Bam-8 fragment (1.0 ug) was mixed with 0.5 ug of pBR327 vector DNA which had been previously digested with both BamHI (2 units) and 0.2 units of calf alkaline phosphatase (CAP) for 1 hour at 37°C; the mixture was deproteinized and resuspended as described in previous examples. The mixed fragments were treated with T4 ligase (2 units), used to transform E. coli C600 recA cells and ampicillin-resistant colonies were selected as described previously. Mini-preps to obtain plasmid DNA were performed on twelve of these clones. The DNA was digested with BamHI to demonstrate the presence of the pBR327 vector and 7.5 kb Bam-8 fragments. One plasmid demonstrating both fragments was designated pMON90. DNA was prepared as described in Example 1.

Twenty-five ug of pMON90 DNA were digested with BglII (25 units, 2 hours, 37°C) the 2.6 kb BglII fragment was purified using NA-45 membrane. To create blunt ends, the fragment (2 ug) was resuspended in 10 ul of 50 mM NaCl, 6.6 mM Tris-HCl pH8, 6.6 mM MgCl$_2$ and 0.5 mM dithiothreitol (Klenow Buffer). The 4 deoxy-nucleoside triphosphates (dATP, dTTP, dCTP, and dGTP) were added to a final concentration of 1 mM and one unit of E. coli large Klenow fragment of DNA polymerase I (New England Biolabs, Beverly, MA) was added. After incubation for 20 minutes at 22°C, the Klenow polymerase was heat inactivated and 10 units of HindIII was added. The HindIII digestion was carried out for 1 hour at 37°C and then the enzyme was heat inactivated. The HindIII-BglII (blunt) fragments (1 ug) were added to 0.25 ug of the 2.2 kb HindIII-PvuII fragment of pBR322 (Bolivar, et al, 1977) which had been generated by HindIII and PvuII digestion then treating with calf alkaline phosphatase as described in previous examples. After ligation using 100 units of T4 DNA ligase, transformation of E. coli LE392 cells and selection of ampicillin-resistant colonies as described in previous examples, nineteen colonies were obtained. Plasmid mini-preps were prepared from twelve colonies and digested with HindIII to determine the size of the recombinant plasmid and with SmaI to determine that the correct fragment had been inserted. One plasmid with the correct structure was designated pMON113, as shown in Figure 6. Plasmid DNA was prepared as described in Example 1.

Example 5: Creation of Plasmid pMON120

Twenty ug of plasmid pMON109 (described in Example 3) were digested with EcoRI and BamHI (20 units each, 2 hours, 37°C) and the 3.4 kb BamHI-EcoRI fragment was purified using NA-45 membrane as described in previous examples. Twenty ug of plasmid pMON41 (described in Example 1) were digested with BamHI and PvuI (20 units each, 2 hours, 37°C) and the 1.5 kb BamHI-PvuI fragment purified using NA-45 membrane as described in previous examples.

Twenty ug of pMON113 DNA (described in Example 4) were digested with PvuI and EcoRI (2 units each, 2 hr, 37°C) and the 3.1 kb PvuI-EcoRI fragment was purified using NA-45 membrane as above. To assemble plasmid pMON120, the 3.1 kb EcoRI-PvuI pMON113 fragment (1.5 ug) was mixed with 1.5 ug of the 3.4 kb EcoRI-BamHI fragment from pMON109. After treatment with T4 ligase (3 units) for 16 hours at 10°C, the ligase was inactivated by heating (10 minutes, 70°C), and 5 units of BamHI was added. Digestion continued for 30 minutes at 37°C at which time the BamHI endonuclease was inactivated by heating as above. Next, 0.75 ug of the 1.5 kb PvuI-BamHI fragment from pMON41 was added along with T4 DNA ligase ( 2 units) and fresh ATP to 0.75 mM final concentration. The final ligase reaction was carried out for 4 hours at 22°C at which time the mixture was used to transform E. coli LE 392 cells with subsequent selection for spectinomycin resistant cells as described previously. Plasmid mini-preps from twelve out of several thousand colonies were screened for plasmids of approximately 8 kb in size containing single sites for BamHI and EcoRI. One plasmid showing the correct structure was designated pMON120, which is shown in Figure 7 with an alternate method of construction. pMON120 DNA was prepared as described in Example 1.

A culture of E. coli containing pMON120 has been deposited with the American Type Culture Collection. This culture has been assigned accession number 39263.

Example 6: Creation of Plasmids pMON128 and pMON129

Plasmid pMON75 (described in detail in a separate application entitled "Chimeric Genes Suitable for Expression in Plant Cells," previously cited) contains a chimeric NOS-NPT II-NOS gene. This plasmid (and pMON128, described below) may be digested by EcoRI and a 1.5 kb fragment may be purified which contains the NOS-NPT II-NOS gene.

Plasmid pMON120 was digested with EcoRI and treated with calf alkaline phosphatase. After phenol deproteinization and ethanol precipitation, the EcoRI-cleaved pMON120 linear DNA was mixed with 0.5 ug of the 1.5 kb EcoRI chimeric gene fragment from pMON75 or 76. The mixture was treated with 2 units of T4 DNA ligase for 1 hour at 22°C. After transformation of E. coli cells and selection of colonies resistant to spectinomycin (50 ug/ml), several thousand colonies appeared. Six of these were picked, grown, and plasmid mini-preps made. The plasmid DNA's were digested with EcoRI to check for the 1.5 kb chimeric gene insert and with BamHI to determine the orientation of the insert. BamHI digestion showned that in pMON128 the chimeric gene was transcribed in the same direction as the intact nopaline synthase gene of pMON120. A culture of E. coli containing pMON128 has been deposited with the American Type Culture Collection. This culture has been assigned accession number 39264. The orientation of the insert in pMON129 was opposite that in pMON128; the appearance of an additional 1.5 kb BamHI fragment in digests of pMON129 showed that plasmid pMON129 carried a tandem duplication of the chimeric NOS-NPT II-NOS gene, as shown in Figure 8.

Example 7: Creation of Co-integrate Plasmid pMON128::pTiB6S3TraC

Plasmid pMON128 (described in Example 6) was transferred to a chloramphenicol resistant Agrobacterium tumefaciens strain GV3111 = C58C1 carrying Ti plasmid pTiB6S3tra[C] (Leemans, et at, 1982) using a tri-parental plate mating procedure, as follows. 0.2 ml of a culture of E. coli carrying pMON128 was mixed with 0.2 ml of a culture of E. coli strain HB101 carrying a pRK2013 plasmid (Ditta, et at, 1980) and 0.2 ml of GV3111 cells. The mixture of cells was cultured in Luria Broth (LB), spread on an LB plate, and incubated for 16 to 24 hours at 30°C to allow plasmid transfer and generation of co-integrate plasmids. The cells were resuspended in 3 ml of 10 mM $MgSO_4$ and 0.2 ml aliquot was then spread on an LB plate containing 25 ug/ml chloramphenicol and 100 ug/ml each of spectinomycin and streptomycin. After incubation for 48 hr at 30°C approximately 10 colonies were obtained. One colony was chosen and grown at 30°C in LB medium containing chloramphenicol, spectinomycin, and streptomycin at the concentrations given above.

A separate type of co-integrate plasmid for use in control experiments was prepared by inserting pMON120 into A. tumefaciens cells, and selecting for cells with co-integrate plasmids using spectinomycin and streptomycin, as described above. Like pMON120, these plasmids do not contain the chimeric NOS-NPT II-NOS gene.

Example 8: Solutions Used in Plant Cell Cultures

The following solutions were used by the Applicants:

|  |  | __per liter__ |
|---|---|---|
| **Enzyme mix:** | Cellulysin | 5 g |
|  | Macerozyme | 0.7 g |
|  | Ampicillin | 0.4 g |
|  | $KH_2PO_4$ | 27.2 mg |
|  | $KNO_3$ | 101 mg |
|  | $CaCl_2$ | 1.48 g |
|  | $MgSO_4 \cdot 7H_2O$ | 246 mg |
|  | KI | 0.16 mg |
|  | $CuSO_4 \cdot 5H_2O$ | 0.025 mg |
|  | Mannitol | 110 g |
| **MS9:** | MS salts(see below) | 4.3 g |
|  | Sucrose | 30.0 g |

```
                         B5 vitamins (see below)  1 ml
                         Mannitol                 90.0 g
                         Phytohormones:
                            Benzyladenine (BA)    0.5 mg
                            2,4-D                 1 mg
         MS-ES           MS salts                 4.3 g
                         Sucrose                  30 g
                         B5 Vitamins              1 ml
                         Mannitol                 30 g
                         Carbenicillin            10 mg
                         Phytohormones:
                            Indole acetic acid    0.1 mg
         MSO:            MS salts                 4.3 g
                         Sucrose                  30.0 g
                         B5 vitamins              1 ml
         Feeder plate    MS salts                 4.3 g
         medium:         Sucrose                  30.0 g
                         B5 vitamins              1 ml
                         Mannitol                 30.0 g
                         Phytohormones:
                            BA                     0.5 mg
         Ms2C            MS salts                 4.3 g
                         Sucrose                  30 g
                         B5 vitamins               1 ml
                         Phytohormones:
                            chlorophenoxyacetic    2 mg
                            acid
         MS104:          MS salts                 4.3 g
                         Sucrose                  30.0 g
                         B5 vitamins              1 ml
                         Phytohormones:
                            BA                     0.1 mg
                            NAA                    1 mg
         MS11:           MS salts                 4.3 g
                         Sucrose                  30.0 g
                         B5 vitamins              1 ml
                         Phytohormones:
                            Zeatin                 1 mg
         B5 Vitamin      myo-inositol             100 g
         stock:          thiamine HCl             10 g
                         nicotinic acid           1 g
                         pyrodoxine HCl           1 g
         Float rinse:    MS salts                 0.43 g
                         Sucrose                  171.2 g
                         PVP-40                   40.0 g
```

MS salts are purchased pre-mixed as a dry powder from Gibco Laboratories, Grand Island, N.Y.

Example 9: Preparation of Protoplasts

Mitchell petunia plants were grown in growth chambers with two or three banks of fluorescent lamps and two banks of incandescent bulbs (about 5,000 lux). The temperature was maintained at a constant 21°C

and the lights were on for 12 hours per day. Plants were grown in a 50/50 mix of Vermiculite and Pro-mix BX (Premier Brands Inc., Canada). Plants were watered once a day with Hoagland's nutrient solution. Tissue was taken from dark green plants with compact, bushy growth. Leaves were sterilized in a solution of 10% commercial bleach and a small amount of detergent or Tween 20 for 20 minutes with occasional agitation. Leaves were rinsed two or three times with sterile distilled water, Thin strips (about 1 mm) were cut from the leaves, perpendicular to the main rib. The strips were placed in the enzyme mix at a ratio of about 1 g tissue to 10 ml enzymes. The dishes were sealed with parafilm, and incubated in the dark or under low, indirect light while gently agitating continuously (e.g., 40 rpm on gyrotary shaker). Enzymic incubations generally were run overnight, about 16-20 hours.

The digestion mixture was sieved through a 68, 74, or 88 um screen to remove large debris and leaf material. The filtrate was spun at 70-100 g for five minutes to pellet the protoplasts. The supernatant was decanted and the pellet was gently resuspended in float rinse solution. This suspension was poured into babcock bottles. The bottles were filled to 2 or 3 cm above the base of the neck. 1 ml of growth medium MS9 was carefully layered on top of the float rinse.

The Babcock bottles were balanced and centrifuged at 500 to 1000 rpm for 10 to 20 minutes. The protoplasts formed a compact band in the neck at the interface. The band was removed with a pipette, taking care not to pick up any excess float rinse. The protoplasts were diluted into MS9. At this point, the protoplasts were washed with MS9 or diluted for plating without washing.

Protoplasts were suspended in MS9 medium at $5 \times 10^4$ per ml, and plated into T-75 flasks, at 6 ml per flask. Flasks were incubated on a level surface with dim, indirect light or in the dark at 26-28°C. On the third day following the removal of the enzymes from the leaf tissue, MSO (medium which does not contain mannitol) was added to each flask, using an amount equal to one-half the original volume. The same amount of MSO is added again on day 4. This reduces the mannitol concentration to about 0.33 M after the first dilution, and about 0.25 M after the second dilution.

Example 10: Co-cultivation with Bacteria

On day 5 following protoplast isolation, five to seven day old tobacco suspension cultures (TXD cells) were diluted (if necessary) with MS2C medium to the point where 1 ml would spread easily over the surface of agar medium in a 100 x 15 mm petri plate [this is a 10 to 15% suspension (w/v)]. The agar medium was obtained by mixing 0.8% agar with MS-ES medium, autoclaving the mixture, and cooling the mixture until it solidifies in the plate. One ml of the TXD suspension was spread over 25 ml of feeder plate medium. An 8.5 cm disc of Whatman #1 filter paper was laid over the TXD feeder cells and smoothed out. A 7 cm disc of the same paper was placed in the center of the larger one.

Separately, aliquots of a culture of A. tumefaciens cells (grown in yeast extract peptone medium) were added to the flasks which contained the plant cells. One set of aliquots contained cells with the pMON128::Ti co-integrate plasmids having chimeric NOS-NPT II-NOS genes. The other set of aliquots contained cells with the pMON120::Ti co-integrate plasmids, which do not have chimeric NOS-NPT II-NOS genes.

The bacteria were added to the flasks to a density of $10^8$ cells/ml. 0.5 ml of the cell mixture was spread in a thin layer on the surface of the 7 cm filter paper disc. The plates were wrapped in parafilm or plastic bags and incubated under direct fluorescent lighting, no more than five plates in a stack.

Within seven days, colonies were discernable. Within 14 days, the 7 cm discs, with colonies adhering to them, were transferred to new MSO agar medium (without feeder cells) containing 500 ug/ml carbenicillin, as well as 50 ug/ml of kanamycin sulfate (Sigma, St. Louis, MO). Within two weeks, vigorously growing green colonies could be observed on the plates which contained plant cells that had been co-cultured with A. tumefaciens strains containing the pMON128 co-integrate NOS-NPT II plasmid. No transformed colonies were detected on plates which contained plant cells that had been co-cultured with A. tumefaciens strains containing the pMON120 co-integrate plasmid. The kanamycin resistant transformants are capable of sustained growth in culture medium containing kanamycin. Southern blotting experiments (as described in E. Southern, J. Mol. Biol. 98: 503 (1975)) confirmed that these cells contain the chimeric NOS-NPT II gene.

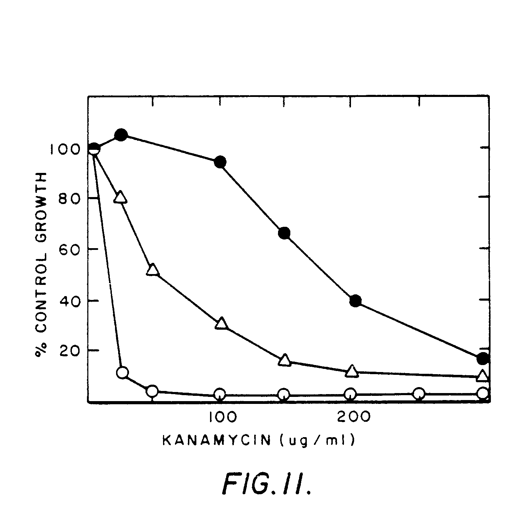

Both sets of transformed cells (and a third set of cells which had been transformed in the same manner by a chimeric gene coding for the enzyme NPT type I) were assayed for resistance to kanamycin. The results are indicated in Figure 11.

Example 11: Regeneration of Transformed Plants

The transformed kanamycin-resistant colonies described in Example 10 contained both tumorous and non-tumorous cells, as described in Figure 9 and the related text. The following procedure was used to isolate non-tumorous transformed cells from tumorous transformed cells, and to regenerate differentiated plant tissue from the non-tumorous cells.

Colonies were grown on MS104 agar medium containing 30 ug/ml kanamycin sulfate and 500 ug/ml carbenicillin until they reached about 1 cm in diameter. Predominantly tumorous colonies appear a somewhat paler shade of green and are more loosely organized than predominantly non-tumorous colonies were removed from the MS104 medium by tweezers and placed upon MS11 medium containing 30 ug/ml kanamycin and 500 ug/ml carbenicillin. As the colonies continued to grow, colonies that appeared pale green and loosely organized were removed and discarded.

MS11 medium contains zeatin, a phytohormone which induces shooting formation in non-tumorous colonies. Several shoots were eventually observed sprouting from kanamycin-resistant colonies. These shoots may be grown to a desired size, and cut off by a sharp blade, and inserted into agar medium without phytohormones, such as MSO, where it may generate roots. If desired, the medium may be supplemented by napthalene acetic acid to induce rooting. The plants may be grown to a desired size in the agar medium, and then transferred into soil. If properly cultivated, such plants will grow to maturity and generate seed. The acquired trait will be inherited by progeny according to classic Mendelian genetics.

References:

A. Bale et al, Mut. Res. 59: 157 (1979)

F. Bolivar, Gene 4: 121 (1978)

A. Braun and H. Wood, Proc. Natl. Acad. Sci. USA 73: 496 (1976)

M. D. Chilton et al , Cell 11: 263 (1977)

M-D Chilton et al, Stadler Symposia, Vol 13, 39-51 (1981) University of Missouri

P.V. Choudary et al, Miami Winter Symposia, Vol 19, (1982) page 514, Academic Press

A. Colman et al, Eur. J. Biochem 91: 303-310 (1978)

T. Currier and E. Nester, J. Bact. 126: 157 (1976)

M. Davey et al, Plant Sci. Lett. 18: 307 (1980)

R. W. Davis et al, Advanced Bacterial Genetics Cold Spring Harbor Laboratory, New York, (1980)

H. De Greve et al, Plasmid 6: 235 (1981)

G. Ditta et al, Proc. Natl. Acad. Sci. USA 77: 7347 (1980)

D. Garfinkel et al, Cell 27: 143 (1981)

S. Hasezawa et al, Mol. Gen. Genet. 182: 206 (1981)

J. Hernalsteens et al, Nature 287: 654 (1980)

D. Ish-Horowicz and J. F. Burke, Nucleic Acids Res. 9: 2989-2998 (1981)

B. Koekman et al, J. Bacteriol. 141: 129 (1979)

F. Krens et al, Nature 296: 72 (1982)

J. Leemans et al, J. Mol. Appl. Genet. 1: 149 (1981)

J. Leemans, H. de Greve et al. Molec. Biology of Plant Tumors, Academic Press (1982), 537-545.

J. Leemans et al, The EMBO J. 1: 147 (1982)

A. L. Lehninger, Biochemistry, 2nd ed. (Worth Publ., 1975)

P. Lurquin, Nucleic Acids. Res. 6: 3773 (1979)

T. Maniatis et al, Molecular Cloning, A Laboratory Manual (Cold Spring Harbor Labs, 1982)

L. Marton et al, Nature 277: 129 (1979)

T. Matzke and M-D Chilton, J. Mol. Appl. Genet. 1: 39 (1981)

J. Messing et al, Nucleic Acids Res. 9: 309 (1981)

J. Messing and J. Vieira, Gene 19: 269-276 (1982)

J. Miller, Experiments in Molecular Genetics, Cold Spring Harbor Laboratory, N.Y. (1972)

N. Murray et al, J. Mol. Biol. 132: 493 (1979)

G. Ooms et al, Plasmid 7: 15 (1982)

L. Otten and R. Schilperoort, Biochim. Biophys Acta 527: 497 (1978)

L. Otten, Mol. Gen. Genet. 183: 209 (1981)

L. W. Ream and M.P. Gordon, Science, Z18, 854-859, (1982)

R. Roberts, Nucleic Acids Res. 10: r117 (1982)

A. Rorsch and R. Schilperoort, Genetic Engineering, 189 Elsevier/North Holland, N.Y. (1978)

J. K. Setlow and A. Hollaender, Genetic Engineering, Principles and Methods (Plenum Press 1979)

X. Soberon et al, Gene 9: 287 (1980)

L. Stryer, Biochemistry, 2nd. ed. (W. H. Freeman and Co., 1981)

M. Thomashow et al, Cell 19: 729 (1980)
B. Vogelstein and D. Gillespie, Proc. Natl. Acad. Sci.: 615-619 (1979)
L. Willmitzer et al, Nature 287: 359 (1980)
L. Willmitzer et al, The EMBO J 1: 139 (1982)
N. Yadev et al, Nature 287: 1 (1980)
F. Yang et al, Mol. Gen. Genet. 177: 707 (1980)
P. Zambryski et al, J. Mol. Appl. Genet. 1: 361 (1982)

**Claims**

1. A co-integrate plasmid for use in transforming plant cells, and produced by recombination by a single crossover event of

   (A) a chimeric plasmid comprising a gene which functions in plants to express an encoded polypeptide, said plasmid comprising in sequence:

   (a) a region of DNA which is homologous to T-DNA located near the left T-DNA border of a tumor-inducing plasmid of Agrobacterium and which is capable of causing in vivo recombination of the chimeric plasmid with said tumor-inducing plasmid of Agrobacterium by a single crossover event;

   (b) a gene comprising a promoter which functions in plant cells, a structural coding sequence encoding a polypeptide expressable in plant cells and a 3' non-translated region encoding a polyadenylation signal, said promoter and polyadenylation signal being operably linked to said structural coding sequence; and

   (c) an Agrobacterium plasmid T-DNA right border sequence which enables the transfer and incorporation of T-DNA into the genome of a plant cell;

   said chimeric plasmid containing no plant tumorigenic genes between and including the region (a), the gene (b) and the border sequence (c), and

   (B) a Ti plasmid capable of transferring the T-DNA region into the genome of a plant cell,

   such that the co-integrate plasmid comprises a T-DNA region having the following elements in sequence

   (i) a left T-DNA border sequence
   (ii) the gene (b) of the chimeric plasmid;
   (iii) at least one right T-DNA border sequence;

   wherein said T-DNA border sequences enable the transfer of the T-DNA region into the genome of a plant cell, and wherein, between and including the left T-DNA border sequence, the gene (b) and the right T-DNA border sequence or (if there is more than one right T-DNA border sequence) between the gene (b) and the right T-DNA border sequence which is nearest to the left T-DNA border sequence, (1) there are no genes which would render a transformed plant cell tumorous or incapable of regeneration into a morphologically normal plant, and (2) there is no duplication of DNA sequences, which duplication could, through homologous recombination, result in loss of the gene (b).

2. A plasmid of Claim 1 in which the polypeptide functions as a selectable marker in transformed plant cells.

3. A plasmid of Claim 2 in which the polypeptide causes the plant cells to be antibiotic resistant.

4. A plasmid of Claim 3 in which the polypeptide is neomycin phosphotransferase.

5. A plasmid of any of Claims 1 to 4 in which the plant tumor inducing plasmid is a wild-type Ti plasmid.

6. A plasmid of any of Claims 1 to 4 in which the plant tumor inducing plasmid is a disarmed Ti plasmid in which the tumorigenic genes and T-DNA right border have been removed.

7. A co-integrate Ti plasmid of Claim 1 having a first gene which causes expression of a polypeptide which functions as a selectable marker in bacteria and a T-DNA region comprising a second gene which causes expression of a polypeptide which functions as a selectable marker gene in the transformed plant cells, and a third gene which causes expression of a third polypeptide in plant cells.

**8.** The plasmid of Claim 7 in which the third gene functions as a scorable marker gene.

**9.** The plasmid of Claim 8 in which the third gene is a chimeric gene.

**10.** The plasmid of either one of Claims 8 and 9 created by a single crossover event between a chimeric plasmid comprising a gene which functions in plants to express an antibiotic resistance marker, said plasmid comprising in sequence:
(a) a region of DNA which is homologous to T-DNA located near the left T-DNA border of a tumor-inducing plasmid of Agrobacterium and which is capable of causing in vivo recombination of the chimeric plasmid with said tumor-inducing plasmid of Agrobacterium by a single crossover event;
(b) a gene comprising a promoter which functions in plant cells, a structural coding sequence encoding an antibiotic resistance marker expressable in plant cells and a 3' non-translated region encoding a polyadenylation signal, said promoter and polyadenylation signal being operably linked to said structural coding sequence; and
(c) an Agrobacterium plasmid T-DNA right border sequence which enables the transfer and incorporation of T-DNA into the genome of a plant cell;
said chimeric plasmid containing no plant tumorigenic genes between and including the region (a), the gene (b) and the border sequence (c);
and a plant tumor inducing plasmid of Agrobacterium.

**11.** The plasmid of Claim 7 in which the second gene imparts antibiotic resistance to plant cells.

**12.** The plasmid of Claim 11 in which the second gene expresses neomycin phosphotransferase.

**13.** The plasmid of Claim 7 which contains no tumorigenic genes.

**14.** A microorganism which contains a chimeric plasmid of Claim 1.

**15.** A microorganism which contains a chimeric plasmid of Claim 7.

**16.** The microorganism of Claim 15 which contains a chimeric plasmid of Claim 12.

**17.** A culture of microorganisms of Claim 14.

**18.** The culture of Claim 17 identified by ATCC number 39266.

**Patentansprüche**

**1.** Cointegrales Plasmid zur Verwendung beim Transformieren von Pflanzenzellen und gebildet durch Rekombination durch einen einzigen Crossover
(A) eines chimären Plasmids umfassend ein Gen, das in Pflanzen zum Exprimieren eines codierten Polypeptids wirkt, welches Plasmid in Sequenz umfaßt:
(a) eine zu T-DNA homologe DNA-Region, die sich nahe der linken T-DNA-Grenze eines tumorinduzierenden Plasmids von Agrobacterium befindet und die imstande ist, in vivo Rekombination des chimären Plasmids mit dem tumorinduzierenden Plasmid von Agrobacterium durch einen einzigen Crossover zu bewirken,
b) ein Gen, umfassend einen in Pflanzenzellen wirkenden Promotor, eine Codierstruktursequenz, die ein in Pflanzenzellen exprimierbares Polypeptid codiert, und eine 3'-nicht-translatierte Region, die ein Polyadenylierungssignal codiert, wobei der Promotor und das Polyadenylierungssignal wirkungsmäßig mit der Codierstruktursequenz verknüpft sind, und
c) eine Agrobacterium-Plasmid-T-DNA-rechte Grenzsequenz, die den Transfer und das Einschleusen von T-DNA in das Genom einer Pflanzenzelle ermöglicht,
welches chimäre Plasmid keine pflanzentumorerzeugenden Gene zwischen und einschließend Region (a), Gen (b) und Grenzsequenz (c) enthält, und
(B) eines Ti-Plasmids, das zum Transferieren der T-DNA-Region in das Genom einer Pflanzenzelle imstande ist,
derart, daß das cointegrale Plasmid eine T-DNA-Region mit folgenden Elementen in Sequenz aufweist:

22

(i) eine linke T-DNA-Grenzsequenz,

(ii) das Gen (b) des chimären Plasmids,

(iii) mindestens eine rechte T-DNA-Grenzsequenz,

wobei die T-DNA-Grenzsequenzen den Transfer der T-DNA-Region in das Genom einer Pflanzenzelle ermöglichen und wobei es zwischen und einschließlich linker T-DNA-Grenzsequenz, Gen (b) und rechter T-DNA-Grenzsequenz oder (wenn es mehr als eine rechte T-DNA-Grenzsequenz gibt) zwischen Gen (b) und rechter T-DNA-Grenzsequenz, welche der linken T-DNA-Grenzsequenz am nächsten ist, (1) keine Gene gibt, die eine transformierte Pflanzenzelle tumorös oder zur Regeneration in eine morphologisch normale Pflanze unfähig machen würden, und (2) keine Duplizierung von DNA-Sequenzen gibt, welche Duplizierung durch homologe Rekombination in Verlust des Gens (b) resultieren könnte.

2. Plasmid nach Anspruch 1, in dem das Polypeptid als Selektionsmarker in transformierten Pflanzenzellen wirkt.

3. Plasmid nach Anspruch 2, in dem das Polypeptid bewirkt, daß die Pflanzenzellen antibiotikumresistent sind.

4. Plasmid nach Anspruch 3, in dem das Polypeptid Neomycinphosphotransferase ist.

5. Plasmid nach einem der Ansprüche 1 bis 4, in dem das pflanzentumorinduzierende Plasmid ein Wildtyp Ti-Plasmid ist.

6. Plasmid nach einem der Ansprüche 1 bis 4, in dem das pflanzentumorinduzierende Plasmid ein entwaffnetes Ti-Plasmid ist, in dem die tumorerzeugenden Gene und die rechte T-DNA-Grenze entfernt wurden.

7. Cointegrales Ti-Plasmid nach Anspruch 1 mit einem ersten Gen, das Expression eines Polypeptids bewirkt, das als Selektionsmarker in Bakterien wirkt, und einer T-DNA-Region, die ein zweites Gen umfaßt, das Expression eines Polypeptids bewirkt, das als Selektionsmarkergen in den transformierten Pflanzenzellen wirkt, und einem dritten Gen, das Expression eines dritten Polypeptids in Pflanzenzellen bewirkt.

8. Plasmid nach Anspruch 7, in dem das dritte Geu als Zählmarkergen wirkt.

9. Plasmid nach Anspruch 8, in dem das dritte Gen ein chimäres Gen ist.

10. Plasmid nach einem der Ansprüche 8 und 9, gebildet durch einen einzigen Crossover zwischen einem chimären Plasmid umfassend ein Gen, das in Pflanzen zum Exprimieren eines Antibiotikumresistenzmarkers wirkt, welches Plasmid in Sequenz umfaßt:

(a) eine zu T-DNA homologe DNA-Region, die sich nahe der linken T-DNA-Grenze eines tumorinduzierenden Plasmids von Agrobacterium befindet und die imstande ist, in vivo Rekombination des chimären Plasmids mit dem tumorinduzierenden Plasmid von Agrobacterium durch einen einzigen Crossover zu bewirken,

b) ein Gen umfassend einen in Pflanzenzellen wirkenden Promotor, eine Codierstruktursequenz, die einen in Pflanzenzellen exprimierbaren Antibiotikumresistenzmarker codiert, und eine 3'-nicht-translatierte Region, die ein Polyadenylierungssignal codiert, wobei der Promotor und das Polyadenylierungssignal wirkungsmäßig mit der Codierstruktursequenz verknüpft sind, und

c) eine Agrobacterium-Plasmid-T-DNA-rechte Grenzsequenz, die den Transfer und das Einschleusen von T-DNA in das Genom einer Pflanzenzelle ermöglicht,

welches chimäre Plasmid keine pflanzentumorerzeugenden Gene zwischen und einschließend Region (a), Gen (b) und Grenzsequenz (c) enthält,

und einem pflanzentumorinduzierenden Plasmid von Agrobacterium.

11. Plasmid nach Anspruch 7, in dem das zweite Gen den Pflanzenzellen Antibiotikumresistenz verleiht.

12. Plasmid nach Anspruch 11, in dem das zweite Gen Neomycinphosphotransferase exprimiert.

EP 0 131 624 B1

**13.** Plasmid nach Anspruch 7, das keine tumorerzeugenden Gene enthält.

**14.** Mikroorganismus, der ein chimäres Plasmid nach Anspruch 1 enthält.

**15.** Mikroorganismus, der ein chimäres Plasmid nach Anspruch 7 enthält.

**16.** Mikroorganismus nach Anspruch 15, der ein chimäres Plasmid nach Anspruch 12 enthält.

**17.** Kultur von Mikroorganismen von Anspruch 14.

**18.** Kultur nach Anspruch 17, identifiziert durch ATCC-Nr. 39266.

**Revendications**

**1.** Plasmide de co-intégrat pour l'utilisation dans la transformation de cellules végétales, et préparé par recombinaison par un enjambement unique de :

(A) un plasmide chimérique comprenant un gène qui fonctionne dans les plantes pour exprimer un polypeptide codé, ce plasmide comprenant successivement:

(a) une région d'ADN qui est homologue à l'ADN-T située au voisinage du bord gauche de l'ADN-T d'un plasmide d'Agrobacterium provoquant des tumeurs et qui est capable de provoquer une recombinaison in vivo du plasmide chimérique avec ce plasmide d'Agrobacterium provoquant des tumeurs par un enjambement unique;

(b) un gène comprenant un promoteur qui fonctionne dans des cellules végétales, une séquence codante structurale codant pour un polypeptide exprimable dans les cellules végétales et une région non traduite 3' codant pour un signal de polyadénylation, ce promoteur et ce signal de polyadénylation étant liés de manière opérationnelle à cette séquence codante structurale; et

(c) une séquence de bord droit d'ADN-T de plasmide d'Agrobactérium qui permet le transfert et l'incorporation de l'ADN-T dans le génome d'une cellule végétale;

ce plasmide chimérique ne contenant pas de gènes tumorigènes pour les plantes entre et y compris la région (a), le gène (b) et la séquence de bord (c), et

(B) un plasmide Ti qui est capable de transférer la région de l'ADN-T dans le génome d'une cellule végétale, tel que le plasmide de co-intégrat comprend une région d'ADN-T ayant successivement les éléments suivants

(i) une séquence de bord gauche d'ADN-T;

(ii) le gène (b) du plasmide chimérique;

(iii) au moins une séquence de bord droit d'ADN-T;

dans lequel ces séquences de bord d'ADN-T permettent le transfert de la région d'ADN-T dans le génome d'une cellule végétale et dans lequel, entre et y compris la séquence de bord d'ADN-T gauche, le gène (b) et la séquence de bord d'ADN-T droit ou (s'il y a plus d'une séquence de bord d'ADN-T droit) entre le gène (b) et la séquence de bord d'ADN-T droit qui est la plus proche de la séquence de bord d'ADN-T gauche, (1) il n'y a pas de gènes qui rendraient une cellule végétale transformée tumorale ou incapable de régénération dans une plante morphologiquement normale, et (2) il n'y a pas de duplication de séquences d'ADN, laquelle duplication pourrait, par recombinaison homologue, conduire à une perte du gène (b).

**2.** Plasmide selon la revendication 1, dans lequel le polypeptide fait office de marqueur sélectionnable dans les cellules végétales transformées.

**3.** Plasmide selon la revendication 2, dans lequel le polypeptide confère aux cellules végétales la résistance aux antibiotiques.

**4.** Plasmide selon la revendication 3, dans lequel le polypeptide est la néomycine phosphotransférase.

**5.** Plasmide selon l'une quelconque des revendications 1 à 4, dans lequel le plasmide provoquant la tumeur de la plante est un plasmide Ti du type sauvage.

**6.** Plasmide selon l'une quelconque des revendications 1 à 4, dans lequel le plasmide provoquant la tumeur végétale est un plasmide Ti désarmé dont les gènes tumorigènes et le bord droit de l'ADN-T

24

ont été éliminés.

**7.** Plasmide Ti de co-intégrat selon la revendication 1, ayant un premier gène qui provoque l'expression d'un polypeptide qui fait fonction de marqueur sélectionnable dans des bactéries et une région d'ADN-T comprenant un second gène qui provoque l'expression d'un polypeptide qui fait office de gène marqueur sélectionnable dans les cellules végétales transformées, et un troisième gène qui provoque l'expression d'un troisième polypeptide dans les cellules végétales.

**8.** Plasmide selon la revendication 7, dans lequel le troisième gène fait office de marqueur cochable.

**9.** Plasmide selon la revendication 8, dans lequel le troisième gène est un gène chimérique.

**10.** Plasmide selon l'une quelconque des revendications 8 et 9, crée par un enjambement unique entre un plasmide chimérique comprenant un gène qui fonctionne dans les plantes pour exprimer un marqueur de résistance aux antibiotique, ce plasmide comprenant successivement :
(a) une région d'ADN qui est homologue de l'ADN-T située au voisinage du bord gauche de l'ADN-T d'un plasmide d'Agrobacterium provoquant des tumeurs et qui est capable de provoquer in vivo la recombinaison du plasmide chimérique avec ce plasmide d'Agrobacterium provoquant des tumeurs par un enjambement unique;
(b) un gène comprenant un promoteur qui fonctionne dans des cellules végétales, une séquence codante structurale codant pour un marqueur de résistance aux antibiotiques exprimable dans des cellules végétales et une région non traduite 3' codant pour un signal de polyadénylation, ce promoteur et ce signal de polyadénylation étant liés de manière opérationnelle à cette séquence codante structurale; et
(c) une séquence de bord droit d'ADN-T de plasmide d'Agrobacterium qui permet le transfert et l'incorporation de l'ADN-T dans le génome d'une cellule végétale;
ce plasmide chimérique ne contenant pas de gènes tumorigènes entre et y compris la région (a), le gène (b) et la séquence de bord (c);
et un plasmide d'Agrobacterium provoquant des tumeurs végétales.

**11.** Plasmide selon la revendication 7, dans lequel le second gène confère aux cellules végétales la résistance aux antibiotiques.

**12.** Plasmide selon la revendication 11, dans lequel le second gène exprime la néomycine phosphotransférase.

**13.** Plasmide selon la revendication 7, qui ne contient pas de gènes tumorigènes.

**14.** Microorganisme qui contient un plasmide chimérique selon la revendication 1.

**15.** Microorganisme qui contient un plasmide chimérique selon la revendication 7.

**16.** Microorganisme selon la revendication 15, qui contient un plasmide chimérique selon la revendication 12.

**17.** Culture de microorganismes selon la revendication 14.

**18.** Culture selon la revendication 17, identifiée par le numéro ATCC 39266.

ISOLATE DNA SEQUENCE WITH MARKER GENE FOR
SELECTION OF A. TUM. CONTAINING COINTEGRATE
PLASMIDS

(pMON 109)

ISOLATE DNA SEQUENCE WITH
T-DNA BORDER FROM TI PLAS-
MID (OPTIONAL: PLANT
SCORABLE MARKER)

(pMON 41)

ISOLATE DNA SEQUENCE WITH
REGION HOMOLOGOUS TO PART
OF T-DNA OF TI PLASMID

(pMON 113)

LIGATE 3 SEQUENCES IN PROPER ORIENTATION

(pMON 120)

INSERT CHIMERIC GENE INTO PLASMID

(pMON 128)

INSERT PLASMID INTO E. COLI;
MATE E. COLI; WITH A. TUMEFACIENS

INSERT PLASMID INTO A. TUMEFACIENS
CONTAINING (DISARMED) TI PLASMID

SELECT A. TUMEFACIENS CELLS WITH
CO-INTEGRATED TI PLASMIDS HAVING
CHIMERIC GENES

CO-CULTIVATE A. TUMEFACIENS WITH
PLANT CELLS; ALLOW TRANSFER OF
CO-INTEGRATE PLASMIDS INTO PLANT
CELLS

CULTIVATE PLANT CELL, IDENTIFY PLANT
CELLS HAVING (EXPRESSION OF) CHIMERIC
GENE OR SCORABLE MARKER

REGENERATE PLANT CELLS INTO PLANTS

*FIG.I.*

26

FIG. 2.

FIG.3.

FIG. 4.

*FIG. 5.*

FIG. 6.

FIG. 7.

FIG. 8.

FIG.9.

FIG.10.

*FIG.11.*